(19) Europäisches Patentamt
European Patent Office
Office européen des brevets

(11) EP 2 334 801 B1

(12) # EUROPÄISCHE PATENTSCHRIFT

(45) Veröffentlichungstag und Bekanntmachung des Hinweises auf die Patenterteilung:
**27.12.2017 Patentblatt 2017/52**

(21) Anmeldenummer: **09783054.1**

(22) Anmeldetag: **15.09.2009**

(51) Int Cl.:
***C12P 7/02*** (2006.01) ***C12P 7/22*** (2006.01)
***C12P 13/00*** (2006.01)

(86) Internationale Anmeldenummer:
**PCT/EP2009/061974**

(87) Internationale Veröffentlichungsnummer:
**WO 2010/031776 (25.03.2010 Gazette 2010/12)**

(54) **VERFAHREN ZUR HERSTELLUNG VON L-PHENYLEPHRIN UNTER VERWENDUNG EINER ALKOHOLDEHYDROGENASE**

PROCESS FOR THE PREPARATION OF L-PHENYLEPHRINE EMPLOYING AN ALCOHOL DEHYDROGENASE

PROCÉDÉ DE PRÉPARATION DE L-PHÉNYLÉPHRINE EN UTILISANT D'UNE ALCOOL DÉSHYDROGÉNASE

(84) Benannte Vertragsstaaten:
**AT BE BG CH CY CZ DE DK EE ES FI FR GB GR HR HU IE IS IT LI LT LU LV MC MK MT NL NO PL PT RO SE SI SK SM TR**

(30) Priorität: **17.09.2008 EP 08164488**

(43) Veröffentlichungstag der Anmeldung:
**22.06.2011 Patentblatt 2011/25**

(73) Patentinhaber: **BASF SE**
**67056 Ludwigshafen am Rhein (DE)**

(72) Erfinder:
- **BREUER, Michael**
  **64285 Darmstadt (DE)**
- **PLETSCH, Andreas**
  **67117 Limburgerhof (DE)**
- **HAUER, Bernhard**
  **67136 Fußgönheim (DE)**
- **SIEGEL, Wolfgang**
  **67117 Limburgerhof (DE)**

(74) Vertreter: **BASF IP Association**
**BASF SE**
**G-FLP-C006**
**67056 Ludwigshafen (DE)**

(56) Entgegenhaltungen:
**EP-A1- 0 735 016** **WO-A1-2007/147897**
**WO-A2-2005/108590**

- **GUY, A. ET AL.: "Selective alpha-Chlorination of Alkyl Aryl Ketones" SYNTHESIS, Nr. 12, Dezember 1982 (1982-12) , Seiten 1018-1020, XP002427346**
- **KAJIGAESHI, S. ET AL.: "alpha-Chlorination of Aromatic Acetyl Derivatives with Benzyltrimethylammonium Dichloroiodate" SYNTHESIS, Nr. 7, Juli 1988 (1988-07), Seiten 545-546, XP002427345**
- **YANG, Y. ET AL.: "Enzymatic ketone reduction: mapping the substrate profile of a short-chain alcohol dehydrogenase (YMR226c) from Saccharomyces cerevisiae" TETRAHEDRON ASYMMETRY, Bd. 18, Nr. 15, 9. August 2007 (2007-08-09), Seiten 1799-1803, XP022243428**
- **DATABASE WPI Week 200957 Thomson Scientific, London, GB; AN 2009-M81855 XP002583156 & CN 101 503 714 A (UNIVERSITY GUANGDONG TECHNOLOGY) 12. August 2009 (2009-08-12)**

Bemerkungen:
Die Akte enthält technische Angaben, die nach dem Eingang der Anmeldung eingereicht wurden und die nicht in dieser Patentschrift enthalten sind.

EP 2 334 801 B1

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

SEQUENCE LISTING

<110> BASF SE

<120> Verfahren zur Herstellung von optisch aktiven Alkoholen

<130> M/49024-PCT

<160> 2

<170> PatentIn version 3.3

<210> 1
<211> 750
<212> DNA
<213> Aromatoleum aromaticum EbN1

<220>
<221> CDS
<222> (1)..(750)

<400> 1

```
atg acg caa aga ctg aag gac aag ctt gca gta att acc ggc ggt gcc      48
Met Thr Gln Arg Leu Lys Asp Lys Leu Ala Val Ile Thr Gly Gly Ala
1               5                   10                  15

aac ggc atc ggg cgg gca att gcg gag cga ttt gcg gtc gaa ggt gcc      96
Asn Gly Ile Gly Arg Ala Ile Ala Glu Arg Phe Ala Val Glu Gly Ala
            20                  25                  30

gac atc gca atc gcg gat ctg gtg ccg gcc ccg gaa gcc gag gca gca     144
Asp Ile Ala Ile Ala Asp Leu Val Pro Ala Pro Glu Ala Glu Ala Ala
        35                  40                  45

atc agg aac ctc ggt cgg cgc gtt ctg acc gtg aag tgc gat gtc tcg     192
Ile Arg Asn Leu Gly Arg Arg Val Leu Thr Val Lys Cys Asp Val Ser
    50                  55                  60

caa cct ggc gac gta gaa gca ttc gga aag cag gtc atc tcc acg ttt     240
Gln Pro Gly Asp Val Glu Ala Phe Gly Lys Gln Val Ile Ser Thr Phe
65                  70                  75                  80

ggt cgc tgc gac atc ctc gtc aac aac gcg gga att tac ccg ctg att     288
Gly Arg Cys Asp Ile Leu Val Asn Asn Ala Gly Ile Tyr Pro Leu Ile
                85                  90                  95

cct ttt gac gag ctg acc ttt gaa cag tgg aag aaa aca ttc gag atc     336
Pro Phe Asp Glu Leu Thr Phe Glu Gln Trp Lys Lys Thr Phe Glu Ile
            100                 105                 110

aac gtc gat tca ggt ttt ctt atg gcg aag gct ttt gtc ccc ggg atg     384
Asn Val Asp Ser Gly Phe Leu Met Ala Lys Ala Phe Val Pro Gly Met
        115                 120                 125

aag agg aac ggg tgg gga cgc atc atc aac ctg act tcg acg aca tat     432
Lys Arg Asn Gly Trp Gly Arg Ile Ile Asn Leu Thr Ser Thr Thr Tyr
    130                 135                 140

tgg cta aag atc gag gcg tat acc cat tac atc agc acg aaa gcg gca     480
Trp Leu Lys Ile Glu Ala Tyr Thr His Tyr Ile Ser Thr Lys Ala Ala
145                 150                 155                 160
```

```
aac ata ggc ttt acc cgc gcc ctt gcc tcg gac ctg ggg aag gac gga      528
Asn Ile Gly Phe Thr Arg Ala Leu Ala Ser Asp Leu Gly Lys Asp Gly
                165                 170                 175

atc act gtt aac gcc atc gcg ccg agc ctt gtc cgc acg gca aca acc      576
Ile Thr Val Asn Ala Ile Ala Pro Ser Leu Val Arg Thr Ala Thr Thr
            180                 185                 190

gaa gct tct gca ttg tcc gcg atg ttc gac gtg ctg cca aac atg ctt      624
Glu Ala Ser Ala Leu Ser Ala Met Phe Asp Val Leu Pro Asn Met Leu
        195                 200                 205

cag gcg att ccg cgt ctt cag gtg ccc ctg gat ctg acg ggc gca gct      672
Gln Ala Ile Pro Arg Leu Gln Val Pro Leu Asp Leu Thr Gly Ala Ala
    210                 215                 220

gcg ttc ctg gct tcc gat gac gcc agt ttt att aca ggc cag acg ctc      720
Ala Phe Leu Ala Ser Asp Asp Ala Ser Phe Ile Thr Gly Gln Thr Leu
225                 230                 235                 240

gcg gtt gat ggc ggt atg gtg aga cac tga                              750
Ala Val Asp Gly Gly Met Val Arg His
                245
```

<210> 2
<211> 249
<212> PRT
<213> Aromatoleum aromaticum EbN1

<400> 2

```
Met Thr Gln Arg Leu Lys Asp Lys Leu Ala Val Ile Thr Gly Gly Ala
1               5                   10                  15

Asn Gly Ile Gly Arg Ala Ile Ala Glu Arg Phe Ala Val Glu Gly Ala
            20                  25                  30

Asp Ile Ala Ile Ala Asp Leu Val Pro Ala Pro Glu Ala Glu Ala Ala
        35                  40                  45

Ile Arg Asn Leu Gly Arg Arg Val Leu Thr Val Lys Cys Asp Val Ser
    50                  55                  60

Gln Pro Gly Asp Val Glu Ala Phe Gly Lys Gln Val Ile Ser Thr Phe
65                  70                  75                  80

Gly Arg Cys Asp Ile Leu Val Asn Asn Ala Gly Ile Tyr Pro Leu Ile
                85                  90                  95

Pro Phe Asp Glu Leu Thr Phe Glu Gln Trp Lys Lys Thr Phe Glu Ile
            100                 105                 110

Asn Val Asp Ser Gly Phe Leu Met Ala Lys Ala Phe Val Pro Gly Met
        115                 120                 125
```

```
Lys Arg Asn Gly Trp Gly Arg Ile Ile Asn Leu Thr Ser Thr Thr Tyr
    130                 135                 140

Trp Leu Lys Ile Glu Ala Tyr Thr His Tyr Ile Ser Thr Lys Ala Ala
145                 150                 155                 160

Asn Ile Gly Phe Thr Arg Ala Leu Ala Ser Asp Leu Gly Lys Asp Gly
                165                 170                 175

Ile Thr Val Asn Ala Ile Ala Pro Ser Leu Val Arg Thr Ala Thr Thr
            180                 185                 190

Glu Ala Ser Ala Leu Ser Ala Met Phe Asp Val Leu Pro Asn Met Leu
        195                 200                 205

Gln Ala Ile Pro Arg Leu Gln Val Pro Leu Asp Leu Thr Gly Ala Ala
    210                 215                 220

Ala Phe Leu Ala Ser Asp Asp Ala Ser Phe Ile Thr Gly Gln Thr Leu
225                 230                 235                 240

Ala Val Asp Gly Gly Met Val Arg His
                245
```

**Beschreibung**

Die vorliegende Erfindung betrifft ein mehrstufiges Verfahren zur Herstellung von substituierten, optisch aktiven Alkoholen, umfassend einen enzymkatalysierten, von einer Alkoholdehydrogenase katalysierten Syntheseschritt. Insbesondere eignet sich das erfindungsgemäße Verfahren zur Herstellung von Phenylephrin, d.h. 3-[(1*R*)-1-Hydroxy-2-methyla mino-ethyl]-phenol.

**Hintergrund der Erfindung:**

Phenylephrin ist ein pharmakologischer Wirkstoff aus der Gruppe der Sympathomimetika und besitzt agonistische Aktivität am $\alpha_1$-adrenergen Rezeptor. Es ist bis auf die fehlende 3-Hydroxylgruppe strukturgleich mit Adrenalin und findet hauptsächlich Anwendung als lokaler Vasokonstriktor. Als Wirkstoff in Nasentropfen wirkt es somit abschwellend auf Schleimhäute. In Augentropfen wirkt es außerdem als Mydriatikum, führt also zu einer Weitstellung der Pupillen.

Die Herstellung von Phenylephrin ist in der Literatur bereits beschrieben. Neben den zahlreichen Verfahren, das gewünschte Produkt als Racemat herzustellen und anschließend durch eine Spaltung mit einem geeigneten chiralen Hilfsmittel in das Produkt zu überführen, sind die Methoden zur stereoselektiven Synthese als bevorzugt anzusehen, da hierbei die unwirtschaftliche Vernichtung des zu 50% anfallenden falschen Enantiomers vermieden werden kann.

Zu den aus dem Stand der Technik bekannten Herstellungsverfahren von L-Phenylephrinhydrochlorid zählt die asymmetrische Hydrierung des prochiralen N-Benzy-N-methyl-2-amino-m-benzyloxyacetophenonhydrochlorids nach Tetrahedron Letters 30 (1989), 367- 370, bzw. Chem. Pharm. Bull. 43 (5) (1995) 738-747.

Achiwa et al. beschreiben in Tetrahedron Letters 30 (1989), 367 - 370 die asymmetrische Hydrierung von 3-Benzyloxy-2-(N-benzyl-N-methyl)-aminoacetophenon-hydrochlorid als Substrat mit Wasserstoff in Gegenwart von $[Rh(COD)Cl]_2$ / (2R,4R)-4-(Dicyclohexylphosphino)-2-(diphenylphosphino-methyl)-N-methyl-aminopyrrolidin als Katalysator. Unmittelbar nach Filtration und Einengung des Reaktionsgemischs wird die benzylische Stickstoff-Schutzgruppe abgespalten und Phenylephrin als Produkt erhalten. Dabei fällt neben dem L-Enantiomeren das D-Enantiomere mit einem Anteil von mindestens 7,5% als Verunreinigung an (85% ee). Für die Reaktion muss der Katalysator, bezogen auf das Substrat, in einem Mol-Verhältnis von 1: 2000 eingesetzt werden. Der Nachteil dieses Verfahrens besteht im wesentlichen darin, dass das erhaltene L-Phenylephrin nicht in wirtschaftlicher Weise auf eine für die Verwendung als Arzneimittel erforderliche Reinheit von mindestens 98% ee aufgereinigt werden kann.

In Chem. Pharm. Bull. 43 (5) (1995) 738 - 747 wird für die asymmetrische Hydrierung ein Mol-Verhältnis von Substrat zu Katalysator von etwa 1000: 1 als bevorzugt angegeben. Das Produkt ist jedoch trotz der Verwendung größerer Mengen Katalysator im asymmetrischen Reaktionsschritt als L-Enantiomeres für pharmazeutische Zwecke ohne aufwendige Reinigungsprozeduren nicht ausreichend rein herstellbar, sondern nur als Mischung mit einem relativ hohen Anteil an D-Enantiomeren als Verunreinigung zugänglich. Auch stellt die relativ lange Reaktionszeit des asymmetrischen Hydrierungsschritts von ca. 20 Stunden gerade für die Herstellung von L-Phenylephrin in industriellem Maßstab einen apparativ sehr aufwendigen und teuren Reaktionsschritt mit einem nicht zu vernachlässigenden Sicherheitsrisiko dar.

Das in WO 00/43345 gelehrte Verfahren erfüllt einige der genannten Bedingungen für eine wirtschaftlich sinnvolle Herstellung von L-Phenylephrin-Hydrochlorid jedoch kann auch hier auf die Verwendung von Schutzgruppen nicht verzichtet werden wodurch das Verfahren weniger wirtschaftlich wird. Weiterhin fällt auch nach diesem Verfahren bei dem stereoselektiven Schritt das gewünschte Produkt nur mit 93% ee an, so dass auch hier eine aufwendige Reinigung nachgeschaltet werden muss.

In Tetrahedron Asymmetry 18 (15) (2007) 1799 - 1803 wurde die Aktivität und Enantioselektivität der Dehydrogenase YMR226c aus *S. cerevisiae* mit einer Reihe von Ketonen untersucht. Es wird beschrieben, dass die Dehydrogenase YMR226c enantioselektive Reduktionen von arylsubstituierten Acetophenonen, $\alpha$-Chloracetophenonen, aliphatischen Ketonen und $\alpha$- und $\beta$-Ketoestern katalysiert.

Die WO 2005/108590 beschreibt ein Verfahren zur Herstellung optisch aktiver Alkohole durch enantioselektive Reduktion unter Verwendung der Phenylethanol-Dehydrogenase aus *Azoarcus sp* EbN1.

**Kurze Beschreibung der Erfindung:**

Aufgabe der vorliegenden Erfindung ist daher die Bereitstellung eines neuartigen Verfahrens zur Herstellung von optisch aktiven Alkoholen, wie L-Phenylephrin, welches im Vergleich zum Stand der Technik wirtschaftlicher durchführbar ist. Insbesondere sollte ein solches verbessertes Verfahren ohne die Verwendung von Schutzgruppen auskommen und eine hohe Stereoselektivität besitzen.

Überraschenderweise konnte obige Aufgabe durch Bereitstellung eines Verfahrens zur Herstellung von substituierten, optisch aktiven Alkoholen der Formel IV wie folgt gelöst werden:

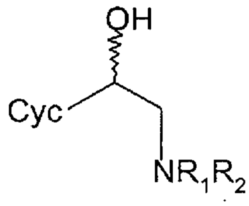

(IV)

worin
Cyc für einen ein- oder mehrkernigen, gesättigten oder ungesättigten, carbocyclischen oder heterocyclischen, gegebenenfalls ein- oder mehrfach substituierten Ring steht, der wenigstens eine freie Hydroxylgruppe trägt, und
$R_1$ und $R_2$ unabhängig voneinander für H oder einen gegebenenfalls ein- oder mehrfach substituierten Alkylrest stehen, oder von Salzen dieser Verbindung,
jeweils in stereoisomerenreiner Form oder als Gemisch von Stereoisomeren,
wobei man

a) ein Keton der Formel

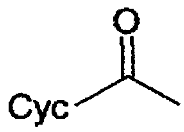

(I)

worin Cyc die oben angegebenen Bedeutungen besitzt,

in Gegenwart eines aliphatischen Alkohols mit einem Halogenierungsmittel zu einer halogenierten Verbindung der Formel II

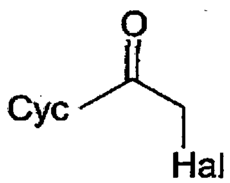

(II)

umsetzt,
worin Cyc die oben angegebenen Bedeutungen besitzt und Hal für ein Halogenatom steht,
b) die so erhaltene Verbindung der Formel II enzymatisch unter Verwendung eines Enzyms, ausgewählt unter Alkoholdehydrogenasen (ADH) (E.C. 1.1.1.1), zum Alkohol der Formel III

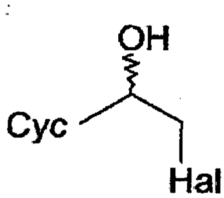

(III)

reduziert,
worin Cyc und Hal die oben angegebenen Bedeutungen besitzen, und
c) den so erhaltenen Alkohol der Formel III mit einem Amin der Formel $HNR_1 R_2$,
worin $R_1$ und $R_2$ die oben angegebenen Bedeutungen besitzen,
zur Verbindung der Formel IV umsetzt.

Darauf aufbauend, ermöglicht die vorliegende Erfindung insbesondere ein überraschend vorteilhaftes Verfahren zur Herstellung des Wirkstoffs Phenylephrin (3-[(1*R*)-1-Hydroxy-2-methylamino-ethyl]-phenol; **4**). Diese bevorzugte Ausführungsform kann durch folgendes Reaktionsschema dargestellt werden:

Schema 1:

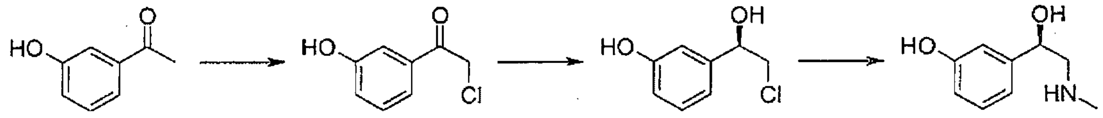

1 2 3 4

Einer der beiden Schlüsselschritte dabei ist die selektive Seitenkettenchlorierung von 3'-Hydroxyacetophenon (3-HAP, **1**) zu 3'-Hydroxy-2-chloracetophenon (HCAP, **2**).

Der zweite Schlüsselschritt betrifft die enantioselektive Reduktion von HCAP (**2**) zu (R)-3-(2-Chlor-1-hydroxyethyl)-phenol (HCPE, **3**), unter Verwendung eines Enzyms, und zwar einer Alkoholdehydrogenase (ADH).

Das erfindungsgemäß bereitgestellte Verfahren unterscheidet sich deutlich in einigen wesentlichen Punkten vom oben diskutierten Stand der Technik.

Die gesamte Synthese kommt nämlich ohne die Verwendung von Schutzgruppen aus wodurch das Verfahren gegenüber dem Stand der Technik wirtschaftlicher ist. Dies ist speziell für die erste Stufe überraschend und unerwartet.

Durch den Einsatz der Alkoholdehydrogenase als Hydrierkatalysator ist ein wirtschaftlicher Zugang zu optisch hochreinem (*R*)-3-(2-Chlor-1-hydroxyethyl)-phenol (HCPE, 3) möglich. Nennenswerte Mengen des unerwünschten Enantiomers werden nicht gebildet. (die %ee-Werte für das gewünschte Enantiomer legen im Bereich von >98%, wie z. B. >99% bis etwa 100%, wie z.B. bis etwa 99,9 %).

Die Reaktion kann (ohne darauf beschränkt zu sein) zudem in einem Zweiphasensystem aus organischem Lösungsmittel und Wasser durchgeführt werden, was zudem ein wirtschaftlicheres Arbeiten ermöglicht. Dabei ist ein vollständiger Umsatz des Ketons zum gewünschten Alkohol möglich. Die Aufarbeitung des Gemisches wird durch die Zweiphasigkeit besonders begünstigt, weil das Produkt von Katalysatorresten (Protein) durch Extraktion getrennt wird. Zudem vermindert die Nutzung der organischen Phase die Exposition des Biokatalysators mit dem niedermolekularen, phenolischen Keton, wodurch eine Inaktivierung und/oder Hemmung des Katalysators vermieden wird.

**Figurenbeschreibung:**

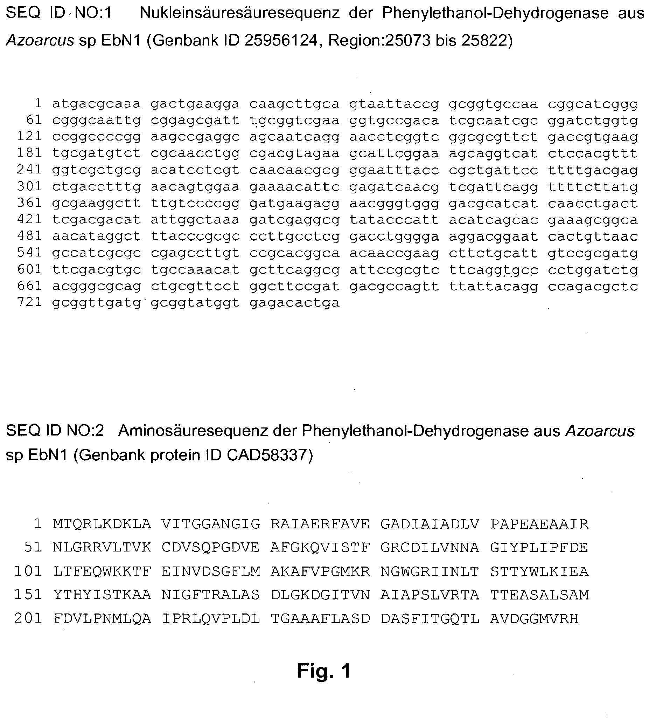

Figur 1 zeigt die Nukleinsäuresäuresequenz bzw. Aminosäuresequenz der Phenylethanol-Dehydrogenase aus (*Azoarcus* sp) *Aromatoleum aromaticum* EbN1 (SEQ ID NO: 1 bzw. 2).

**Detaillierte Beschreibung der Erfindung:**

**1. Bevorzugte Ausführungsformen**

Ein erster Gegenstand der Erfindung betrifft ein Verfahren zur Herstellung von substituierten, optisch aktiven Alkoholen der Formel IV

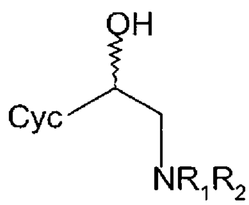

(IV)

worin

Cyc für einen ein- oder mehrkernigen, insbesondere einkernigen, 4 bis 7-gliedrigen, insbesondere 5- oder 6-gliedrigen, gesättigten oder ungesättigten, insbesondere ungesättigten, vor allem aromatischen, carbocyclischen oder heterocyclischen, insbesondere carbocyclischen, Ring steht, der wenigstens eine freie Hydroxylgruppe trägt, und gegebenenfalls ein- oder mehrfach substituiert ist, wobei im Falle eines 6-gliedrigen Rings die Hydroxygruppe(n) insbesondere in meta-Position zur Aminogruppentragenden Seitenkette von Cyc stehen; und

$R_1$ und $R_2$ unabhängig voneinander für H oder gleiche oder verschiedene, gegebenenfalls ein- oder mehrfach substituierte Alkylreste stehen;

oder von Salzen dieser Verbindung, wie z.B. Säureadditionssalzen von insbesondere anorganischen Säuren, wie HCl; jeweils in stereoisomerenreiner Form, wie z.B. der (R)- oder (S)-Form, oder als Gemisch von Stereoisomeren, wie z.B. Racematen,

wobei man

a) ein Keton der Formel I

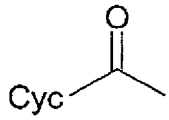
(I)

worin Cyc die oben angegebenen Bedeutungen besitzt,
in Gegenwart eines aliphatischen Alkohols halogeniert, wie insbesondere chloriert, und zwar insbesondere mit Sulfurylchlorid, zu einer halogenierten, insbesondere chlorierten, Verbindung der Formel II umsetzt

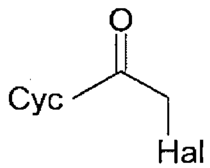
(II)

worin Cyc die oben angegebenen Bedeutungen besitzt und Hal für ein Halogenaton, wie z.B. F, Br oder insbesondere Cl steht;

b) die so erhaltene Verbindung der Formel II, gegebenenfalls nach vorheriger Isolierung oder Anreicherung, enzymatisch unter Verwendung eines Enzyms, ausgewählt unter Alkoholdehydrogenasen (ADH) (E.C. 1.1.1.1) zum Alkohol der Formel III reduziert

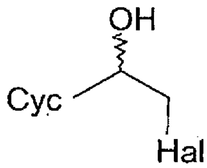
(III)

worin Cyc und Hal die oben angegebenen Bedeutungen besitzen; und

c) den so erhaltenen Alkohol der Formel III, gegebenenfalls nach vorheriger Isolierung oder Anreicherung, mit einem Amin der Formel $HNR_1R_2$, worin $R_1$ und $R_2$ die oben angegebenen Bedeutungen besitzen, zur Verbindung der Formel IV umsetzt und diese gegebenenfalls aus dem Reaktionsgemisch, gegebenenfalls in stereoisomerenreiner Form, isoliert.

Die zur Synthese verwendeten Ketone obiger Formel I sind an sich bekannte Verbindungen und unter Anwendung allgemein bekannter organischer Syntheseverfahren zugänglich.

Insbesondere erfolgt die Umsetzung der Stufe a) in Gegenwart von 1 bis 10, 2 bis 8 oder 3 bis 5 Moläquivalenten des aliphatischen Alkohol je Mol Keton der Formel I.

Geeignete aliphatische Alkohole sind insbesondere Mono- oder Polyole mit 1 bis 6, insbesondere 1 bis 4 Kohlenstoffatomen und 1 bis 5, insbesondere 1 bis 3 Hydroxylgruppen, insbesondere Monoole mit 1 bis 4 Kohlenstoffatiomen, wie z.B. Methanol, Ethanol, n-Propanol, n-Butanol; oder längerkettige Monoole, wie n-Pentanol und n-Hexanol, oder Polyole, wie Propandiol, Butan-1,4-diol, Pentan-1,5-diol, Hexan-1,6-diol oder Pentan-1,3,5-triol; und isomere Formen dieser genannten Alkohole.

Die chemische Umsetzung von Stufe c) kann insbesondere in Lösung in einem offenkettigen oder zyklischen Ether erfolgen. Geeignete Ether sind insbesondere MTBE, Methyl-THF, Dioxan und vor allem THF.

Stufe b) der erfindungsgemäßen Reaktion wird durch wenigstens ein Enzym, ausgewählt unter Alkoholdehydrogenasen (ADH) (E.C. 1.1.1.1) katalysiert. Dabei sind die ADHs, beispielsweise ausgewänlt unter Alkoholdehydrogenasen aus Mikroorganismen der Gattung *Aromatoleum* (*Azoarcus*), insbesondere aus dem Bakterium *Aromatoleum Aromaticum* EbN1.

Beispielsweise ist das Enzym für die Durchführung von Stufe b) ausgewählt unter Enzymen, welche eine Polypeptidsequenz besitzen, die ausgewählt ist unter

(i) SEQ ID NO: 2 oder
(ii) Sequenzen, worin bis zu 25 %, wie z.B. 1 bis 24 %, 2 bis 20 %, 3 bis 15 % der 4 bis 10 %, der Aminosäurereste gegenüber SEQ ID NO: 2 durch Addition, Deletion, Insertion, Substitution, Inversion oder einer Kombination davon verändert sind, und/oder die noch mindestens 50 %, wie z.B. mindestens 60, 70, 80, 90, 95, 96, 97, 98, 99, 100 oder mehr als 100 %, wie z.B. die 1 bis 20-fache, oder 2 bis 10-fache oder 3 bis 5-fache Aktivität, der enzymatischen Aktivität eines Enzyms gemäß SEQ ID NO:2 aufweisen.

Gemäß einer weiteren Ausgestaltung erfolgt die Umsetzung von Stufe b) unter Zugabe von Reduktionsäquivalenten, insbesondere NADH oder NADPH, und gegebenenfalls unter gleichzeitiger oder zeitlich versetzter Regenerierung der bei der Umsetzung verbrauchten Reduktionsäquivalente.

Die Regeneration kann dazu in an sich bekannter Weise enzymatisch, elektrochemisch oder elektroenzymatisch erfolgen (Biotechnology Progress, 2005, 21, 1192; Biocatalysis and Biotransformation, 2004, 22, 89; Angew. Chem Int. Ed Engl., 2001, 40, 169; Biotechnol Bioeng, 2006, 96, 18; Biotechnol Adv., 2007, 25, 369; Angew.Chem Int.Ed Engl., 2008, 47, 2275; Current Opinion in Biotechnology, 2003, 14, 421; Current Opinion in Biotechnology, 2003, 14, 583). Insbesondere erfolgt die Regeneration enzymatisch, und das regenerierende Enzym ist ausgewählt unter ADH (EC.1.1.1.1) und von ADH verschiedenen Dehydrogenasen, wie insbesondere Glucosedehydrogenasen (EC 1.1.1.47), Formiatdehydrogenasen (EC 1.2.1.2 bzw. EC 1.2.1.43), und Phosphitdehydrogenasen (EC 1.20.1.1) und vorzugsweise in Gegenwart eines so genannten "Opferalkohols, wie z.B. Butan- oder Pentan-2-ol, welche bei der enzymatischen Regeneration der Reduktionsäquivalente verbraucht d.h. oxidiert wird.

Insbesondere kann die Umsetzung von Stufe b) entweder in Gegenwart eines Mikroorganismus, der die ADH natürlich oder rekombinant exprimiert, oder in Gegenwart einer davon abgeleiteten, d.h. aus den Zellen gewonnenen, die ADH enthaltende Fraktion oder eines aus den Zellen gewonnenen Zellextraktes, oder in Gegenwart des reinen oder im wesentlichen reinen Enzyms erfolgen. Die erfindungsgemäß verwendeten Enzyme (in Reinform, in angereicherter Form, oder als enzymhaltiger Zellextrakt) werden zudem in an sich bekannter Weise, gelöst, dispergiert oder an einem Träger immobilisiert eingesetzt.

Beispielsweise erfolgt die Umsetzung von Stufe b) in Gegenwart eines Mikroorganismus, der ausgewählt ist unter Bakterien der Familien Enterobacteriaceae, Pseudomonadaceae, Bacillaceae, Rhizobiaceae, Lactobacillaceae, Streptomycetaceae, Rhodococcaceae, Rhodocyclaceae und Nocardiaceae, oder in Gegenwart einer oder eines davon abgeleiteten Fraktion oder Extraktes. Beispiele geeigneter Gattungen umfassen insbesondere Escherichia, Streptomyces, Corynebacterium und Bacillus. Beispiele geeigneter Arten sind insbesondere E. coli.

Insbesondere kann der Mikroorganismus ein rekombinanter Mikroorganismus sein, der mit einem Nukleinsäurekonstrukt transformiert ist, welcher für eine ADH gemäß obiger Definition kodiert. Gegebenenfalls kann der eingesetzte rekombinante Mikroorganismus zusätzlich eine von ADH verschiedene, exogene oder endogene Dehydrogenase gemäß obiger Definition exprimieren, um die Kofaktorregenerierung zu unterstützen.

In einer weiteren Ausgestaltung kann die Umsetzung von Stufe b) in einem zweiphasigen flüssigen Reaktionsmedium durchgeführt werden. Dazu verwendet man z.B. ein wässrig-organisches Reaktionsmedium, wobei sowohl das Edukt der Formel II als auch das Produkt der Formel III in der organischen Phase besser löslich sind als in der wässrigen Phase wie z.B. eine wässrig-etherische Phase, oder z.B. Wasser/Heptan- und Wasser/Hexan-Phasen.

Ein weiterer hierin beschriebener Gegenstand betrifft ein Verfahren zur Herstellung einer Verbindung der allgemeinen Formel II,

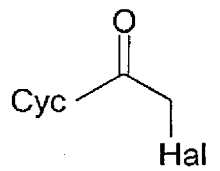
(II)

worin Cyc und Hal die oben angegebenen Bedeutungen besitzen,
wobei man ein Keton der Formel I

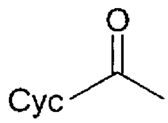

(I)

worin Cyc die oben angegebenen Bedeutungen besitzt, halogeniert, insbesondere chloriert, insbesondere in Gegenwart eines aliphatischen Alkohols mit einem geeigneten Halogenierungsmittel, wie insbesondere Sulfurylchlorid, zur halogenierten, insbesondere chlorierten Verbindung der Formel II umsetzt.

Dabei erfolgt die Umsetzung der Stufe a) insbesondere in Gegenwart von 1 bis 10, wie z.B. 2 bis 8 oder 3 bis 5, Moläquivalenten Alkohol je Mol Keton der Formel I.

Ein weiterer hierein beschriebener Gegenstand betrifft ein Verfahren zur Herstellung einer Verbindung der Formel III

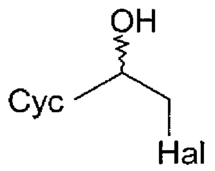

(III)

worin Cyc und Hal die oben angegebenen Bedeutungen besitzen; wobei man eine Verbindung der allgemeinen Formel II

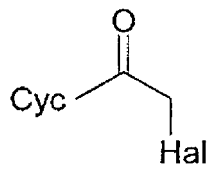

(II)

worin Cyc und Hal die oben angegebenen Bedeutungen besitzen, enzymatisch zum Alkohol der Formel III reduziert. Dabei führt man die enzymatische Reaktion wie oben definiert durch.

Erfindungsgemäß steht Cyc insbesondere für einen einkernigen, carbocyclischen oder heterocyclischen 4-, 5- oder 6-gliedrigen, aromatischen Ring, der wenigstens eine HO-Gruppe trägt, wie insbesondere für einen 3-Hydroxyphenylrest. Hal steht insbesondere für ein Chloratom.

Ein weiterer Gegenstand der Erfindung betrifft die Verwendung einer Alkoholdehydrogenase nach obiger Definition oder eines dieses Enzym produzierenden Mikroorganismus gemäß obiger Definition zur Herstellung von Verbindungen der FormelIV, insbesondere zur Herstellung von (3-[(1*R*)-1-Hydroxy-2-methylamino-ethyl]-phenol).

## 2. Definitionen

### 2.1 Allgemeine Begriffe

Werden keine anderen Angaben gemacht, so gelten folgende allgemeine Bedeutungen:

"Optisch aktiv" sind erfindungsgemäß Verbindungen mit mindestens einem Asymmetriezentrum im Molekül.

Eine "freie Hydroxylgruppe" bedeutet erfindungsgemäß, dass diese nicht in derivatisierter Form, wie z.B. als Ester oder Ethergruppe, vorliegt.

Unter stereoisomerenreinen oder enantiomerenreinen Produkten, wie (3-[(1*R*)-1-Hydroxy-2-methylamino-ethyl]-phenol oder (*R*)-3-(2-Chlor-1-hydroxyethyl)-phenol, sind erfindungsgemäß Enantiomere zu verstehen, die eine Enantiomerenanreicherung zeigen. Insbesondere werden im erfindungsgemäßen Verfahren Enantiomerenreinheiten von mindestens 90 %ee, bevorzugt von mindestens 95 %ee, besonders bevorzugt von mindestens 98 %ee, ganz besonders bevorzugt mindestens 99 %ee oder mehr, erreicht.

Die "Enantiomerenreinheit" wir definiert über den Parameter

$$ee\% = [X_A-X_B]/[ X_A+X_B]*100,$$

worin $X_A$ und $X_B$ für den Molenbruch der Enantiomere A bzw B stehen.

"Enzymatisch" erfolgt eine Umsetzung entweder in Gegenwart reiner Enzyme, angereicherter Enzyme oder ganzer Zellen.

### 2.2 Spezielle chemische Bedeutungen

"Ein- oder mehrkernige" Reste sind Reste, die eine oder mehrere cyclischen Gruppen, umfassen, wobei im Falle mehrkerniger Reste diese cyclischen Gruppen miteinander direkt oder über übliche Brückengruppen verbunden oder miteinander kondensiert sein können.

"Carbocyclische" Reste umfassen ausschließlich Ring-Kohlenstoffatome; "Heterocyclische" Reste umfassen zudem ein oder mehrere, wie z.B. 1, 2 oder 3, gleiche oder verschiedene Ring-heteroatome, wie N, O oder S.

Diese carbo- oder heterocyclischen Ringe umfassen insbesondere 3 bis 12, vorzugsweise 4, 5 oder 6 Ring-Kohlenstoffatomen. Als Beispiele können genannt werden Cyclopropyl, Cyclobutyl, Cyclopenty, Cyclohexyl, Cycloheptyl, die ein- oder mehrfach ungesättigten Analoga davon, wie Cyclobutenyl, Cyclopentenyl, Cyclohexenyl, Cycloheptenyl, Cyclohexadienyl, Cycloheptadienyl; sowie 5- bis 7-gliedrige gesättigte oder ein oder mehrfach ungesättigte heterocyclische Reste mit 1 bis 4 Heteroatomen, die ausgewählt sind unter O, N und S, wobei der Heterocyclus gegebenenfalls mit einem weiteren Heterocyclus oder Carbocyclus kondensiert sein kann. Insbesondere sind zu nennen heterocyclische Reste, abgeleitet von Pyrrolidin, Tetrahydrofuran, Piperidin, Morpholin, Pyrrol, Furan, Thiophen, Pyrazol, Imidazol, Oxazol, Thiazol, Pyridin, Pyran, Pyrimidin, Pyridazin, Pyrazin, Cumaron, Indol und Chinolin.

Nichtlimitierende Beispiele geeigneter "Substituenten" sind ausgewählt unter Halogen, OH, -SH, $-NO_2$, Niedrigalkyl, Niedrigalkenyl, Niedrigalkoxy und Aryl.

"Halogen" steht für Fluor, Chlor, Brom oder Jod , insbesondere Fluor, Brom oder Chlor.

"Niedrigalkyl" steht für geradkettige oder verzweigte Alkylreste 1 bis 6 C-Atomen, wie Methyl, Ethyl, i- oder n-Propyl, n-, i-, sec.- oder tert.-Butyl, n-Pentyl oder 2-MethylButyl, n-Hexyl, 2-Methyl-pentyl, 3-Methyl-pently, 2-Ethyl-butyl.

"Niedrigalkenyl" steht für die ein- oder mehrfach, vorzugsweise einfach oder zweifach ungesättigten Analoga oben genannter Alkylreste mit 2 bis 6 Kohlenstoffatomen, wobei die Doppelbindung in beliebiger Position der Kohlenstoffkette liegen kann.

"Niedrigalkoxy" steht für die Sauerstoff-terminierten Analoga obiger Alkylreste.

"Aryl" steht für einen ein- oder mehrkernigen, vorzugsweise ein- oder zweikernigen, gegebenenfalls substituierten aromatischen Rest, insbesondere für Phenyl oder für ein über eine beliebige Ringposition gebundenes Naphthyl, wie 1- oder 2-Naphthyl. Diese Arylreste können gegebenenfalls 1 oder 2 gleiche oder verschiedene Substituenten tragen, ausgewählt unter Halogen, Niedrigalkyl, Niedrigalkoxy gemäß obiger Definition oder Trifluormethyl.

Beispiele für geeignete Cyc-Reste sind Phenyl, Naphthyl, 2-Thienyl, 3-Thienyl; 2-Furanyl, 3-Furanyl; 2-Pyridyl, 3-Pyridyl oder 4-Pyridyl; 2-Thiazolyl, 4-Thiazolyl oder 5-Thiazolyl; 4-Methyl-2-thienyl, 3-Ethyl-2-thienyl, 2-Methyl-3-thienyl, 4-Propyl-3-thienyl, 5-n-Butyl-2-thienyl, 4-Methyl-3-thienyl, 3-Methyl-2-thienyl; 3-Chlor-2-thienyl, 4-Brom-3-thienyl, 2-Iod-3-thienyl, 5-Iod-3-thienyl, 4-Fluor-2-thienyl, 2-Brom-3-thienyl, und 4-Chlor-2-thienyl, welche zusätzlich wenigstens eine Hydroxyl-Ringsubstituenten tragen.

### 3. Spezielle Ausgestaltungen des erfindungsgemäßen Verfahrens

Weitere Ausgestaltungen der Erfindung werden unter Bezugnahme auf die in obigem Schema 1 dargestellte mehrstufige Reaktion im Folgenden erläutert. Davon ausgehend liegen Abwandlungen dieses konkret beschriebenen Verfahrens im Bereich fachmännischen Könnens.

### 3.1 Selektive Seitenkettenchlorierung von 3'-Hydroxyacetophenon

Der prinzipielle Einsatz von Sulfurylchlorid zur $\alpha$-Chlorierung von Ketonen ist an sich bekannt und beispielsweise in D.P. Wyman et al., J. Org. Chem. Vol. 29, 1964, Seiten 1956 bis 1960 beschrieben.

US 4,310,702 und D. Masilamani et al., J. Org. Chem., Vol. 46, 1981, Seiten 4486 bis 4489 berichten, dass der Einsatz von Sulfurylchlorid zur Chlorierung von Ketonen im Allgemeinen zu einer Mischung aus einfach- und mehrfach chlorierten Ketonen und somit zu unerwünschten Nebenprodukten führt. Zur Lösung des Problems lehren die Schriften den Einsatz von Alkoholen oder Ethern als Moderator. Weiterhin lehrt diese Publikation die Umsetzung von Phenol mit Sulfurylchlorid die zunächst zum entsprechenden Sulfonsäureester und anschließend zu verschiedenen Chlorphenolen führt. Auch die US 5,710,341, welche die Herstellung von $\alpha$-Chloralkylarylketonen durch Chlorierung des entsprechenden

Ketons mit Sulfurylchlorid betrifft, lehrt den Einsatz von aliphatischen Alkoholen zur Erhöhung der Selektivität zum Wunschprodukt, also dem mono-α-chlorierten Keton.

Es wurde nun überraschender Weise gefunden, daß unter den in US 5,710,341 gelehrten Bedingungen die Umsetzung des für die Synthese von Phenylephrin vorteilhaft einzusetzenden 3-Hydroxyacetophenon eine Chlorierung nahezu ausschließlich zum korrespondierenden α-Chloralkylarylketonen führt. Zur Steuerung der Selektivität setzt man der Reaktion dabei 1-10 Äquivalente eines Alkohols ($C_1$-$C_{10}$) zu, besonders bevorzugt verwendet man zwischen 3 und 5 Äquivalente des Alkohols. Weiterhin wird die Reaktion in einem unter den Reaktionsbedingungen inerten Lösungsmittel wie beispielsweise Aromaten, Ether, Ester und halogenierte Lösungsmittel durchgeführt die mit Wasser nicht mischbar sind. Bevorzugt wird die Durchführung in Estern und halogenierten Lösungsmitteln, besonders bevorzugt in Ethylacetat oder Dichlormethan.

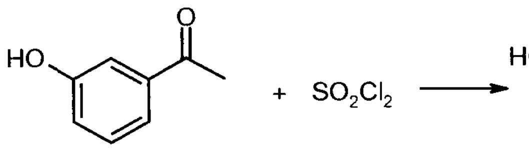

1 2

Diese Tatsache ist für den Fachmann überaschend, da eine Umsetzung der im Molekül vorhandenen phenolischen Funktionalität analog der von D. Masilamani gelehrten Weise eine Bildung der entsprechenden Chlorphenole erwarten ließ. Die Umsetzung kann dabei zweckmäsigerweise ohne die Verwendung einer Schutzgruppe durchgeführt werden.

### 3.2. Enantioselektive Hydrierung von 3'-Hydroxy-2-chloracetophenon

Die Reduktion von **2** wird durch ein Enzym katalysiert. Es handelt sich um die Alkoholdehydrogenase EbN1 aus (*Azoarcus* sp.) *Aromatoleum aromaticum* EbN1, die im speziellen Fall rekombinant in *Escherichia coli* bereitgestellt wird.

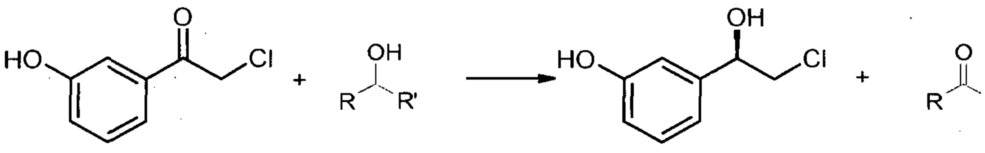

2 3

Es ist bekannt, dass sich Dehydrogenasen als Biokatalysatoren zur Herstellung optisch aktiver Hydroxyverbindungen eignen. Es handelt sich um gut charakterisierte Biokatalysatoren, die bereits in einer Reihe von technischen Prozessen eingesetzt werden (Angew.Chem.Int.Ed., 2004, 43, 788; Tetrahedron, 2004, 60, 633; Chiral catalysis - asymmetric hydrogenation supplement to Chemistry Today, 2004, 22, 26; Current Opinion in Chemical Biology, 2004, 8, 120; Organic Process Research & Development, 2002, 6, 558; Tetrahedron: Asymmetry, 2003, 14, 2659; Chiral catalysis - asymmetric hydrogenation supplement to Chemistry Today, 2004, 22, 43).

Dehydrogenasen setzen Ketone bzw. Aldehyde in die entsprechenden sekundären oder primären Alkohole um; prinzipiell ist die Reaktion reversibel. Sie katalysieren den enantioselektiven Hydridtransfer auf das prochirale C-Atom der Carbonylverbindung.

Die Hydrid-Ionen werden von sogenannten Kofaktoren wie z.B. NADPH oder NADH (reduziertes Nicotinamid-Adenindinucleotidphosphat bzw. reduziertes NicotinamidAdenindinucleotid). Da es sich hierbei um sehr teure Verbindungen handelt, werden sie nur in katalytischen Mengen der Reaktion zugegeben. Die reduzierten Kofaktoren werden während der Reaktion durch eine gleichzeitig stattfindende, zweite Redoxreaktion regeneriert. Je nach der thermodynamischen und kinetischen Bedingungen der Gesamtreaktion können preiswerte sekundäre Alkohole (sogenannte "Opferalkohole") wie zum Beispiel *i*-Propanol als letztendlicher Hydriddonor der Reaktion auftreten, wie es aus der Meerwein-Ponndorf-Verley-Reaktion bekannt ist. Oft können Keton-Reduktion und Opferalkohol-Oxidation vom gleichen Biokatalysator durchgeführt werden (Substrat-Kopplung).

Alternativ kann ein zweiter Katalysator verwendet werden, um die verbrauchten Kofaktoren zu regenerieren. Bekannte Beispiele dafür sind Formiat-Dehydrogenase, Glucosedehydrogenase oder Phosphitdehydrogenase, die aus der Oxidation von Formiat, Glucose bzw. Phosphit Hydridionen aus NAD oder NADP übertragen. (Biocatalysis and

Biotransformation, 2004, 22, 89; Applied Microbiology and Biotechnology, 1997, 48, 699; Bioscience Biotechnology and Biochemistry, 1998, 62, 167; Methods Enzymol., 1987, 136, 9; Ann. N. Y.Acad. Sci. , 1984, 434, 91; FEBS Journal, 2005, 272, 3816; Applied Microbiology and Biotechnology, 2003, 61, 133)

Die Reduktionsäquivalente der hier betrachteten Reaktion stammen entweder aus *i*-Propanol (oder einem anderen sekundären sog. 'Opferalkohol') das zu Aceton oxidiert wird, oder aus Glucose, die in einer parallelen Reaktion zu Gluconolacton oxidiert wird. Während die Oxidation vieler Opferalkohole durch dasselbe Enzym möglich ist, das auch die Reduktion von **2** zu *R*-**3** durchführt, muss für die Oxidation von Glucose als zweites Enzym Glucosedehydrogenase zugeführt werden.

Alternativ kann statt der Glucosedehydrogenase auch ein anderes Regenerationssystem, wie zum Beispiel die Phosphit-Dehydrogenase (Biotechnol Bioeng, 2006, 96, 18) oder eine elektrochemische Kofaktorregeneration (Angew.Chem Int.Ed Engl., 2001, 40, 169), (Angewandte Chemie Int.Ed.Engl., 1999, 29, 388) eingesetzt werden.

Geeignete Biokatalysator für die Herstellung von ***R*-3** sind bereits in folgenden Patentanmeldungen der BASF SE beschreiben: (DE 2004022686, EP 2005004872, WO 2005108590) bzw. (EP 06123814, WO2008055988 A3).

### 3.3 Herstellung von L-Phenylephrin

Den Abschluss dieses neuen Verfahrens zur Herstellung von L-Phenylephrin und seinen Salzen bildet die Umsetzung der nach der Reduktion erhaltenen Komponente **3** mit Methylamin zum gewünschten Produkt.

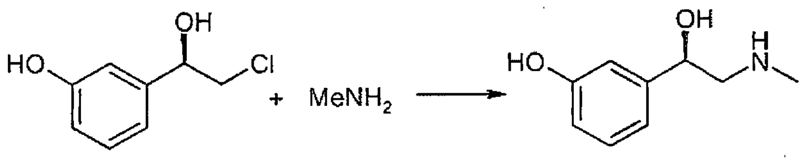

**3** **4**

Diese gelingt in vielen verschiedenen unter den Reaktionbedingungen inerten Lösungsmitteln wie z.B. Wasser, Alkoholen oder Ethern. Besonders bevorzugt sind dabei die Ether in denen sich das Ausgangsmaterial **3** zu einem hohen Anteil löst um in wirtschaftlich sinnvollen Konzentrationen arbeiten zu können. Besonders bevorzugt wird hierfür THF verwendet. Nach der Reaktion kann L-Phenylephrin als Base und in Form seiner Salze zum Beispiel aber nicht ausschließlich nach dem in WO 00/43345 gelehrten Verfahren erhalten werden.

### 4. Weitere Ausgestaltungen der Erfindung

4.1 Alkoholdehydrogenasen

Das eingesetzte Enzym. ist ausgewählt unter Alkoholdehydrogenasen (E.C. 1.1.1.1). Erfindungsgemäß ist es ausgewählt unter Alkoholdehydrogenasen (E.C. 1.1.1.1).

Ohne darauf beschränkt zu sein sind solche Enzyme bevorzugt aus Mikroorganismen der Gattungen *Aromatoleum* (zuweilen auch bezeichnet als *Azoarcus*) zugänglich, wie z.B. *Aromatoleum aromaticum, insbesondere Stamm EbN1.*

Bevorzugte Enzyme mit ADH-Aktivität umfassen eine Aminosäuresequenz gemäß SEQ ID NO: 2.

Erfindungsgemäß mit umfasst sind ebenfalls "funktionale Äquivalente" der konkret offenbarten ADHs und die Verwendung dieser in den erfindungsgemäßen Verfahren.

"Funktionale Äquivalente" oder Analoga der konkret offenbarten Enzyme sind im Rahmen der vorliegenden Erfindung davon verschiedene Polypeptide, welche weiterhin die gewünschte biologische Aktivität, wie z.B. Substratspezifität, besitzen. So versteht man beispielsweise unter "funktionalen Äquivalenten" Enzyme, die 3'-Hydroxy-2-chloracetophenon **2** zum entsprechenden R-Alkohol (R)-3-(2-Chlor-1-hydroxyethyl) phenol **3** reduzieren und die mindestens 50 %, besonders bevorzugt 75 %, ganz besonders bevorzugt 90 % der Aktivität eines Enzyms, umfassend eine der unter SEQ ID NO:2 aufgeführten Aminosäuresequenz, aufweist.

Unter "funktionalen Äquivalenten" versteht man erfindungsgemäß insbesondere auch Mutanten, welche in wenigstens einer Sequenzposition der oben genannten Aminosäuresequenzen eine andere als die konkret genannte Aminosäure aufweisen aber trotzdem eine der oben genannten biologischen Aktivitäten besitzen. "Funktionale Äquivalente" umfassen somit die durch eine oder mehrere Aminosäure-Additionen, - Substitutionen, -Deletionen und/oder -Inversionen erhältlichen Mutanten, wobei die genannten Veränderungen in jeglicher Sequenzposition auftreten können, solange sie zu einer Mutante mit dem erfindungsgemäßen Eigenschaftsprofil führen. Funktionale Äquivalenz ist insbesondere auch dann gegeben, wenn die Reaktivitätsmuster zwischen Mutante und unverändertem Polypeptid qualitativ übereinstimmen, d.h. beispielsweise gleiche Substrate mit unterschiedlicher Geschwindigkeit umgesetzt werden.

"Funktionale Äquivalente" im obigen Sinne sind auch "Präkursoren" der beschriebenen Polypeptide sowie "funktionale Derivate" und "Salze" der Polypeptide.

"Präkursoren" sind dabei natürliche oder synthetische Vorstufen der Polypeptide mit der gewünschten biologischen Aktivität. Ebenfalls beschrieben sind natürliche oder synthetische Vorstufen der Polypeptide ohne die gewünschte biologische Aktivität.

Unter dem Ausdruck "Salze" versteht man sowohl Salze von Carboxylgruppen als auch Säureadditionssalze von Aminogruppen der erfindungsgemäßen Proteinmoleküle. Salze von Carboxylgruppen können in an sich bekannter Weise hergestellt werden und umfassen anorganische Salze, wie zum Beispiel Natrium-, Calcium-, Ammonium-, Eisen- und Zinksalze, sowie Salze mit organischen Basen, wie zum Beispiel Aminen, wie Triethanolamin, Arginin, Lysin, Piperidin und dergleichen. Säureadditionssalze, wie zum Beispiel Salze mit Mineralsäuren, wie Salzsäure oder Schwefelsäure und Salze mit organischen Säuren, wie Essigsäure und Oxalsäure sind ebenfalls von der Erfindung umfasst.

"Funktionale Derivate" erfindungsgemäßer Polypeptide können an funktionellen Aminosäure-Seitengruppen oder an deren N- oder C-terminalen Ende mit Hilfe bekannter Techniken ebenfalls hergestellt werden. Derartige Derivate umfassen beispielsweise aliphatische Ester von Carbonsäuregruppen, Amide von Carbonsäuregruppen, erhältlich durch Umsetzung mit Ammoniak oder mit einem primären oder sekundären Amin; N-Acylderivate freier Aminogruppen, hergestellt durch Umsetzung mit Acylgruppen; oder O-Acylderivate freier Hydroxygruppen, hergestellt durch Umsetzung mit Acylgruppen.

"Funktionale Äquivalente" umfassen natürlich auch Polypeptide welche aus anderen Organismen zugänglich sind, sowie natürlich vorkommende Varianten. Beispielsweise lassen sich durch Sequenzvergleich Bereiche homologer Sequenzregionen festlegen und in Anlehnung an die konkreten Vorgaben der Erfindung äquivalente Enzyme ermitteln.

"Funktionale Äquivalente" umfassen ebenfalls Fragmente, vorzugsweise einzelne Domänen oder Sequenzmotive, der erfindungsgemäßen Polypeptide, welche z.B. die gewünschte biologische Funktion aufweisen.

"Funktionale Äquivalente" sind außerdem Fusionsproteine, welche eine der oben genannten Polypeptidsequenzen oder davon abgeleitete funktionale Äquivalente und wenigstens eine weitere, davon funktionell verschiedene, heterologe Sequenz in funktioneller N- oder C-terminaler Verknüpfung (d.h. ohne gegenseitigen wesentliche funktionelle Beeinträchtigung der Fusionsprofieinteile) aufweisen. Nichtlimitierende Beispiele für derartige heterologe Sequenzen sind z.B. Signalpeptide oder Enzyme.

Erfindungsgemäß mit umfasste "funktionale Äquivalente" sind Homologe zu den konkret offenbarten Proteinen. Diese besitzen wenigstens 75% insbesondere wenigsten 85 %, wie z.B. wie z.B. 90, 91, 92, 93, 94, 95, 96, 97, 98 oder 99%, Homologie zu einer der konkret offenbarten Aminosäuresequenzen.Eine prozentuale Homologie eines erfindungsgemäßen homologen Polypeptids bedeutet insbesondere prozentuale Identität der Aminosäurereste bezogen auf die Gesamtlänge einer der hierin konkret beschriebenen Aminosäuresequenzen.

Unter "Identität" zwischen zwei Sequenzen wird insbesondere die Identität der Reste über die jeweils gesamte Sequenzlänge verstanden, insbesondere die Identität, die durch Vergleich mit Hilfe der Vector NTI Suite 7.1 (Vector NTI Advance 10.3.0, Invitrogen Corp.) (bzw. Software der Firma Informax (USA) unter Anwendung der Clustal Methode (Higgins DG, Sharp PM. Fast and sensitive multiple sequence alignments on a microcomputer. Comput Appl. Biosci. 1989 Apr;5(2):151-1) unter Einstellung folgender Parameter berechnet wird:

| Multiple alignment parameter: | |
|---|---|
| Gap opening penalty | 10 |
| Gap extension penalty | 0,05 |
| Gap separation penalty range | 8 |
| Gap separation penalty | off |
| % identity for alignment delay | 40 |
| Residue specific gaps | off |
| Hydrophilic residue gap | off |
| Transition weighing | 0 |

| Pairwise alignment parameter: | |
|---|---|
| FAST algorithm | off |
| K-tuple size | 1 |
| Gap penalty | 3 |
| Window size | 5 |

(fortgesetzt)

Number of best diagonals 5

Im Falle einer möglichen Proteinglykosylierung umfassen erfindungsgemäße "funktionale Äquivalente" Proteine des oben bezeichneten Typs in deglykosylierter bzw. glykosylierter Form sowie durch Veränderung des Glykosylierungsmusters erhältliche abgewandelte Formen.

Homologe der erfindungsgemäßen Proteine oder Polypeptide können durch Mutagenese erzeugt werden, z.B. durch Punktmutation oder Verkürzung des Proteins.

Homologe der erfindungsgemäßen Proteine können durch Screening kombinatorischer Banken von Mutanten, wie z.B. Verkürzungsmutanten, identifiziert werden. Beispielsweise kann eine variegierte Bank von Protein-Varianten durch kombinatorische Mutagenese auf Nukleinsäureebene erzeugt werden, wie z.B. durch enzymatisches Ligieren eines Gemisches synthetischer Oligonukleotide. Es gibt eine Vielzahl von Verfahren, die zur Herstellung von Banken potentieller Homologer aus einer degenerierten Oligonukleotidsequenz verwendet werden können. Die chemische Synthese einer degenerierten Gensequenz kann in einem DNA-Syntheseautomaten durchgeführt werden, und das synthetische Gen kann dann in einen geeigneten Expressionsvektor ligiert werden. Die Verwendung eines degenerierten Gensatzes ermöglicht die Bereitstellung sämtlicher Sequenzen in einem Gemisch, die den gewünschten Satz an potentiellen Proteinsequenzen kodieren. Verfahren zur Synthese degenerierter Oligonukleotide sind dem Fachmann bekannt (z.B. Narang, S.A. (1983) Tetrahedron 39:3; Itakura et al. (1984) Annu. Rev. Biochem. 53:323; Itakura et al., (1984) Science 198:1056; Ike et al. (1983) Nucleic Acids Res. 11:477).

Im Stand der Technik sind mehrere Techniken zum Screening von Genprodukten kombinatorischer Banken, die durch Punktmutationen oder Verkürzung hergestellt worden sind, und zum Screening von cDNA-Banken auf Genprodukte mit einer ausgewählten Eigenschaft bekannt. Diese Techniken lassen sich an das schnelle Screening der Genbanken anpassen, die durch kombinatorische Mutagenese erfindungsgemäßer Homologer erzeugt worden sind. Die am häufigsten verwendeten Techniken zum Screening großer Genbanken, die einer Analyse mit hohem Durchsatz unterliegen, umfassen das Klonieren der Genbank in replizierbare Expressionsvektoren, Transformieren der geeigneten Zellen mit der resultierenden Vektorenbank und Exprimieren der kombinatorischen Gene unter Bedingungen, unter denen der Nachweis der gewünschten Aktivität die Isolation des Vektors, der das Gen kodiert, dessen Produkt nachgewiesen wurde, erleichtert. Recursive-Ensemble-Mutagenese (REM), eine Technik, die die Häufigkeit funktioneller Mutanten in den Banken vergrößert, kann in Kombination mit den Screeningtests verwendet werden, um Homologe zu identifizieren (Arkin und Yourvan (1992) PNAS 89:7811-7815; Delgrave et al. (1993) Protein Engineering 6(3):327-331).

4.2 Kodierende Nukleinsäuresequenzen

Die Begriffe "exprimieren" oder "Überexpression" beschreiben im Kontext der Erfindung die Produktion bzw. Erhöhung der intrazellulären Aktivität eines oder mehrerer Enzyme in einem Mikroorganismus, die durch die entsprechende DNA kodiert werden. Dazu kann man beispielsweise ein Gen in einen Organismus einbringen, ein vorhandenes Gen durch ein anderes Gen ersetzen, die Kopienzahl des Gens bzw. der Gene erhöhen, einen starken Promotor verwenden oder ein Gen verwenden, das für ein entsprechendes Enzym mit einer hohen Aktivität kodiert und man kann gegebenenfalls diese Maßnahmen kombinieren.

Hierin beschrieben sind insbesondere Nukleinsäuresequenzen, die für ein Enzym mit ADH-Aktivität kodieren. Bevorzugt sind Nukleinsäuresequenzen umfassend eine Sequenz gemäß SEQ ID NO:1; oder von den Aminosäuresequenzen gemäß SEQ ID NO.: 2 abgeleitete Nukleinsäuresequenzen.

Alle hierin erwähnten Nukleinsäuresequenzen (einzel- und doppelsträngige DNA- und RNA-Sequenzen, wie z.B. cDNA und mRNA) sind in an sich bekannter Weise durch chemische Synthese aus den Nukleotidbausteinen, wie beispielsweise durch Fragmentkondensation einzelner überlappender, komplementärer Nukleinsäurebausteine der Doppelhelix herstellbar. Die chemische Synthese von Oligonukleotiden kann beispielsweise, in bekannter Weise, nach der Phosphoamiditmethode (Voet, Voet, 2. Auflage, Wiley Press New York, Seiten 896-897) erfolgen. Die Anlagerung synthetischer Oligonukleotide und Auffüllen von Lücken mit Hilfe des Klenow-Fragmentes der DNA-Polymerase und Ligationsreaktionen sowie allgemeine Klonierungsverfahren werden in Sambrook et al. (1989), Molecular Cloning: A laboratory manual, Cold Spring Harbor Laboratory Press, beschrieben.

Hierin beschrieben sind auch Nukleinsäuresequenzen (einzel- und doppelsträngige DNA- und RNA-Sequenzen, wie z.B. cDNA und mRNA), kodierend für eines der obigen Polypeptide und deren funktionalen Äquivalenten, welche z.B. unter Verwendung künstlicher Nukleotidanaloga zugänglich sind.

Beschrieben sind sowohl isolierte Nukleinsäuremoleküle, welche für Polypeptide bzw. Proteine oder biologisch aktive Abschnitte davon kodieren, als auch Nukleinsäurefragmente, die z.B. zur Verwendung als Hybridisierungssonden oder Primer zur Identifizierung oder Amplifizierung von kodierender Nukleinsäuren verwendet werden können.

Die Nukleinsäuremoleküle können zudem untranslatierte Sequenzen vom 3'- und/oder 5'-Ende des kodierenden Genbereichs enthalten

Beschrieben sind auch die zu den konkret beschriebenen Nukleotidsequenzen komplementären Nukleinsäuremoleküle oder ein Abschnitt davon.

Die hierin beschriebenen Nukleotidsequenzen ermöglichen die Erzeugung von Sonden und Primern, die zur Identifizierung und/oder Klonierung von homologer Sequenzen in anderen Zelltypen und Organismen verwendbar sind. Solche Sonden bzw. Primer umfassen gewöhnlich einen Nukleotidsequenzbereich, der unter "stringenten" Bedingungen (siehe unten) an mindestens etwa 12, vorzugsweise mindestens etwa 25, wie z.B. etwa 40, 50 oder 75 aufeinanderfolgende Nukleotide eines Sense-Stranges einer hierin beschriebenen Nukleinsäuresequenz oder eines entsprechenden Antisense-Stranges hybridisiert.

Ein "isoliertes" Nukleinsäuremolekül wird von anderen Nukleinsäuremolekülen abgetrennt, die in der natürlichen Quelle der Nukleinsäure zugegen sind und kann überdies im wesentlichen frei von anderem zellulären Material oder Kulturmedium sein, wenn es durch rekombinante Techniken hergestellt wird, oder frei von chemischen Vorstufen oder anderen Chemikalien sein, wenn es chemisch synthetisiert wird.

Ein hierin beschriebenes Nukleinsäuremolekül kann mittels molekularbiologischer Standard-Techniken und der erfindungsgemäß bereitgestellten Sequenzinformation isoliert werden. Beispielsweise kann cDNA aus einer geeigneten cDNA-Bank isoliert werden, indem eine der konkret offenbarten vollständigen Sequenzen oder ein Abschnitt davon als Hybridisierungssonde und Standard-Hybridisierungstechniken (wie z.B. beschrieben in Sambrook, J., Fritsch, E.F. und Maniatis, T. Molecular Cloning: A Laboratory Manual. 2. Aufl., Cold Spring Harbor Laboratory, Cold Spring Harbor Laboratory Press, Cold Spring Harbor, NY, 1989) verwendet werden. Überdies lässt sich ein Nukleinsäuremolekül, umfassend eine der offenbarten Sequenzen oder ein Abschnitt davon, durch Polymerasekettenreaktion isolieren, wobei die Oligonukleotidprimer, die auf der Basis dieser Sequenz erstellt wurden, verwendet werden. Die so amplifizierte Nukleinsäure kann in einen geeigneten Vektor kloniert werden und durch DNA-Sequenzanalyse charakterisiert werden. Die erfindungsgemäßen Oligonukleotide können ferner durch Standard-Syntheseverfahren, z.B. mit einem automatischen DNA-Synthesegerät, hergestellt werden.

Hierin beschriebene Nukleinsäuresequenzen, wie SEQ ID No: 1 oder Derivate davon, Homologe oder Teile dieser Sequenzen, lassen sich beispielsweise mit üblichen Hybridisierungsverfahren oder der PCR-Technik aus geeigneten Mikroorganismen, z.B. über genomische oder cDNA-Banken, isolieren. Diese DNA-Sequenzen hybridisieren unter Standardbedingungen mit den hierin beschriebenen Sequenzen. Zur Hybridisierung werden vorteilhaft kurze Oligonukleotide verwendet. Es können aber auch längere Fragmente der hierin beschriebenen Nukleinsäuren oder die vollständigen Sequenzen für die Hybridisierung verwendet werden. Je nach der verwendeten Nukleinsäure (Oligonukleotid, längeres Fragment oder vollständige Sequenz) oder je nach dem, welche Nukleinsäureart DNA oder RNA für die Hybridisierung verwendet werden, variieren diese Standardbedingungen. So liegen beispielsweise die Schmelztemperaturen für DNA:DNA-Hybride ca 10°C niedriger als die von DNA:RNA-Hybriden gleicher Länge.

Unter Standardbedingungen sind beispielsweise je nach Nukleinsäure Temperaturen zwischen 42 und 58 °C in einer wäßrigen Pufferlösung mit einer Konzentration zwischen 0,1 bis 5 x SSC (1 X SSC = 0,15 M NaCl, 15 mM Natriumcitrat, pH 7,2) oder zusätzlich in Gegenwart von 50% Formamid wie beispielsweise 42 °C in 5 x SSC, 50% Formamid zu verstehen. Vorteilhafterweise liegen die Hybridisierungsbedingungen für DNA:DNA-Hybride bei 0,1 x SSC und Temperaturen zwischen etwa 20 °C bis 45°C, bevorzugt zwischen etwa 30 °C bis 45 °C. Für DNA:RNA-Hybride liegen die Hybridisierungsbedingungen vorteilhaft bei 0,1 x SSC und Temperaturen zwischen etwa 30°C bis 55 °C, bevorzugt zwischen etwa 45 °C bis 55 °C. Diese angegebenen Temperaturen für die Hybridisierung sind beispielhaft kalkulierte Schmelztemperaturwerte für eine Nukleinsäure mit einer Länge von ca. 100 Nukleotiden und einem G + C-Gehalt von 50 % in Abwesenheit von Formamid. Die experimentellen Bedingungen für die DNA-Hybridisierung sind in einschlägigen Lehrbüchern der Genetik, wie beispielsweise Sambrook et al., "Molecular Cloning", Cold Spring Harbor Laboratory, 1989, beschrieben und lassen sich nach dem Fachmann bekannten Formeln beispielsweise abhängig von der Länge der Nukleinsäuren, der Art der Hybride oder dem G + C-Gehalt berechnen. Weitere Informationen zur Hybridisierung kann der Fachmann folgenden Lehrbüchern entnehmen: Ausubel et al. (eds), 1985, Current Protocols in Molecular Biology, John Wiley & Sons, New York; Hames and Higgins (eds), 1985, Nucleic Acids Hybridization: A Practical Approach, IRL Press at Oxford University Press, Oxford; Brown (ed), 1991, Essential Molecular Biology: A Practical Approach, IRL Press at Oxford University Press, Oxford.

Hierin beschrieben sind auch Derivate der konkret offenbarten oder ableitbaren Nukleinsäuresequenzen.

So können weitere hierin beschriebene Nukleinsäuresequenzen z.B. von SEQ ID NO:1 abgeleitet sein und sich davon durch Addition, Substitution, Insertion oder Deletion einzelner oder mehrerer Nukleotide unterscheiden, aber weiterhin für Polypeptide mit dem gewünschten Eigenschaftsprofil kodieren.

Hierin beschrieben sind auch solche Nukleinsäuresequenzen, die sogenannte stumme Mutationen umfassen oder entsprechend der Codon-Nutzung eins speziellen Ursprungs- oder Wirtsorganismus, im Vergleich zu einer konkret genannten Sequenz verändert sind, ebenso wie natürlich vorkommende Varianten, wie z.B. Spleißvarianten oder Allelvarianten, davon.

Beschrieben sind ebenso durch konservative Nukleotidsubstutionen (d.h. die betreffende Aminosäure wird

durch eine Aminosäure gleicher Ladung, Größe, Polarität und/oder Löslichkeit ersetzt) erhältliche Sequenzen.

Hierin beschrieben sind auch die durch Sequenzpolymorphismen von den konkret offenbarten Nukleinsäuren abgeleiteten Moleküle. Diese genetischen Polymorphismen können zwischen Individuen innerhalb einer Population aufgrund der natürlichen Variation existieren. Diese natürlichen Variationen bewirken üblicherweise eine Varianz von 1 bis 5 % in der Nukleotidsequenz eines Gens.

Unter Derivaten der hierin beschriebenen Nukleinsäuresequenz mit der Sequenz SEQ ID NO: 1 sind beispielsweise Allelvarianten zu verstehen, die mindestens 40 % Homologie auf der abgeleiteten Aminosäureebene, bevorzugt mindestens 60 % Homologie, ganz besonders bevorzugt mindestens 80 % Homologie über den gesamten Sequenzbereich aufweisen (bezüglich Homologie auf Aminosäureebene sei auf obige Ausführungen zu den Polypeptiden verwiesen auf). Über Teilbereiche der Sequenzen können die Homologien vorteilhaft höher liegen.

Weiterhin sind unter Derivate auch Homologe der Nukleinsäuresequenzen, insbesondere der SEQ ID NO: 1, beispielsweise pilzliche oder bakterielle Homologe, verkürzte Sequenzen, Einzelstrang-DNA oder RNA der kodierenden und nichtkodierenden DNA-Sequenz, zu verstehen. So besitzten z.B. Homologe zur der SEQ ID NO: 1 auf DNA-Ebene eine Homologie von mindestens 40 %, bevorzugt von mindestens 60 %, besonders bevorzugt von mindestens 70 %, ganz besonders bevorzugt von mindestens 80 % über den gesamten in SEQ ID NO: 1 angegebenen DNA-Bereich.

Außerdem sind unter Derivaten beispielsweise Fusionen mit Promotoren zu verstehen. Die Promotoren, die den angegebenen Nukleotidsequenzen vorgeschalten sind, können durch ein oder mehrere Nukleotidaustausche, Insertionen, Inversionen und/oder Deletionen verändert sein, ohne dass aber die Funktionalität bzw. Wirksamkeit der Promotoren beeinträchtigt sind. Des weiteren können die Promotoren durch Veränderung ihrer Sequenz in ihrer Wirksamkeit erhöht oder komplett durch wirksamere Promotoren auch artfremder Organismen ausgetauscht werden.

Unter Derivaten sind auch Varianten zu verstehen, deren Nukleotidsequenz im Bereich von -1 bis -1000 Basen stromaufwärts vor dem Startkodon oder 0 bis 1000 Basen stromabwärts nach dem Stopkodon so verändert wurden, dass die Genexpression und/oder die Proteinexpression verändert, bevorzugt erhöht wird.

Weiterhin beschrieben sind auch Nukleinsäuresequenzen, welchen mit oben genannten kodierenden Sequenzen unter "stringenten Bedingungen" hybridisieren. Diese Polynukleotide lassen sich bei der Durchmusterung von genomischen oder cDNA-Banken auffinden und gegebenenfalls daraus mit geeigneten Primern mittels PCR vermehren und anschließend beispielsweise mit geeigneten Sonden isolieren. Darüber hinaus können hierin beschriebene Polynukleotide auch auf chemischem Wege synthetisiert werden. Unter dieser Eigenschaft versteht man die Fähigkeit eines Poly- oder Oligonukleotids unter stringenten Bedingungen an eine nahezu komplementäre Sequenz zu binden, während unter diesen Bedingungen unspezifische Bindungen zwischen nichtkomplementären Partnern unterbleiben. Dazu sollten die Sequenzen zu 70-100%, vorzugsweise zu 90-100%, komplementär sein. Die Eigenschaft komplementärer Sequenzen, spezifisch aneinander binden zu können, macht man sich beispielsweise in der Northern- oder Southern-Blot-Technik oder bei der Primerbindung in PCR oder RT-PCR zunutze. Üblicherweise werden dazu Oligonukleotide ab einer Länge von 30 Basenpaaren eingesetzt. Unter stringenten Bedingungen versteht man beispielsweise in der Northern-Blot-Technik die Verwendung einer 50 - 70 °C, vorzugsweise 60 - 65 °C warmen Waschlösung, beispielsweise 0,1x SSC-Puffer mit 0,1% SDS (20x SSC: 3M NaCl, 0,3M Na-Citrat, pH 7,0) zur Elution unspezifisch hybridisierter cDNA-Sonden oder Oligonukleotide. Dabei bleiben, wie oben erwähnt, nur in hohem Maße komplementäre Nukleinsäuren aneinander gebunden. Die Einstellung stringenter Bedingungen ist dem Fachmann bekannt und ist z:B. in Ausubel et al., Current Protocols in Molecular Biology, John Wiley & Sons, N.Y. (1989), 6.3.1-6.3.6. beschrieben.

4.3 Verwendete Konstrukte

Es werden außerdem Expressionskonstrukte verwendet, enthaltend unter der genetischen Kontrolle regulativer Nukleinsäuresequenzen eine für ein erfindungsgemäßes Enzym kodierende Nukleinsäuresequenz; sowie Vektoren, umfassend wenigstens eines dieser Expressionskonstrukte.

Vorzugsweise umfassen solche Konstrukte 5'-stromaufwärts von der jeweiligen kodierenden Sequenz einen Promotor und 3'-stromabwärts eine Terminatorsequenz sowie gegebenenfalls weitere übliche regulative Elemente, und zwar jeweils operativ verknüpft mit der kodierenden Sequenz.

Unter einer "operativen Verknüpfung" versteht man die sequentielle Anordnung von Promotor, kodierender Sequenz, Terminator und gegebenenfalls weiterer regulativer Elemente derart, dass jedes der regulativen Elemente seine Funktion bei der Expression der kodierenden Sequenz bestimmungsgemäß erfüllen kann. Beispiele für operativ verknüpfbare Sequenzen sind Targeting-Sequenzen sowie Enhancer, Polyadenylierungssignale und dergleichen. Weitere regulative Elemente umfassen selektierbare Marker, Amplifikatiorissignale, Replikationsursprünge und dergleichen. Geeignete regulatorische Sequenzen sind z.B. beschrieben in Goeddel, Gene Expression Technology: Methods in Enzymology 185, Academic Press, San Diego, CA (1990).

Unter einem eingesetztenen Nukleinsäurekonstrukt sind insbesondere die ADH mit Sequenz SEQ ID NO: 1 und die Derivate und Homologen davon sowie die von SEQ ID NO: 1 ableitbaren Nukleinsäuresequenzen zu verstehen, die mit einem oder mehreren Regulationssignalen vorteilhafterweise zur Steuerung, z.B. Erhöhung, der Genexpression

operativ oder funktionell verknüpft wurden.

Zusätzlich zu diesen Regulationssequenzen kann die natürliche Regulation dieser Sequenzen vor den eigentlichen Strukturgenen noch vorhanden sein und gegebenenfalls genetisch verändert worden sein, so dass die natürliche Regulation ausgeschaltet und die Expression der Gene erhöht wurde. Das Nukleinsäurekonstrukt kann aber auch einfacher aufgebaut sein, das heißt es wurden keine zusätzlichen Regulationssignale vor die kodierende Sequenz (wie z.B. SEQ ID NO: 1 oder seine Homologen) insertiert und der natürliche Promotor mit seiner Regulation wurde nicht entfernt. Stattdessen wird die natürliche Regulationssequenz so mutiert, dass keine Regulation mehr erfolgt und die Genexpression gesteigert wird.

Ein bevorzugtes Nukleinsäurekonstrukt enthält vorteilhafterweise auch eine oder mehrere der schon erwähnten "Enhancer" Sequenzen, funktionell verknüpft mit dem Promotor, die eine erhöhte Expression der Nukleinsäuresequenz ermöglichen. Auch am 3'-Ende der DNA-Sequenzen können zusätzliche vorteilhafte Sequenzen inseriert werden, wie weitere regulatorische Elemente oder Terminatoren. Die hierin beschriebenen Nukleinsäuren können in einer oder mehreren Kopien im Konstrukt enthalten sein. Im Konstrukt können noch weitere Marker, wie Antibiotikaresistenzen oder Auxotrophien komplementierende Gene, gegebenenfalls zur Selektion auf das Konstrukt enthalten sein.

Vorteilhafte Regulationssequenzen für das Verfahren sind beispielsweise in Promotoren wie cos-, tac-, trp-, tet-, trp-tet-, lpp-, lac-, lpp-lac-, $lacI^{q-}$' T7-, T5-, T3-, gal-, trc-, ara-, rhaP ($rhaP_{BAD}$)SP6-, lambda-$P_R$- oder im lambda-$P_L$-Promotor enthalten, die vorteilhafterweise in gram-negativen Bakterien Anwendung finden. Weitere vorteilhafte Regulationssequenzen sind beispielsweise in den gram-positiven Promotoren amy und SPO2, in den Hefe- oder Pilzpromotoren ADC1, MFalpha , AC, P-60, CYC1, GAPDH, TEF, rp28, ADH enthalten. In diesem Zusammenhang sind auch die Promotoren der Pyruvatdecarboxylase und der Methanoloxidase, beispielsweiseaus Hansenula vorteilhaft. Es können auch künstliche Promotoren für die Regulation verwendet werden.

Das Nukleinsäurekonstrukt wird zur Expression in einem Wirtsorganismus vorteilhafterweise in einen Vektor, wie beispielsweise einem Plasmid oder einem Phagen inseriert, der eine optimale Expression der Gene im Wirt ermöglicht. Unter Vektoren sind außer Plasmiden und Phagen auch alle anderen dem Fachmann bekannten Vektoren, also z.B. Viren, wie SV40, CMV, Baculovirus und Adenovirus, Transposons, IS-Elemente, Phasmide, Cosmide, und lineare oder zirkuläre DNA zu verstehen. Diese Vektoren können autonom im Wirtsorganismus repliziert oder chromosomal repliziert werden. Geeignete Plasmide sind beispielsweise in E. coli pLG338, pACYC184, pBR322, pUC18, pUC19, pKC30, pRep4, pHS1, pKK223-3, pDHE19.2, pHS2, pPLc236, pMBL24, pLG200, pUR290, $pIN\text{-}III^{113}$-B1, gt11 oder pBdCl, in Streptomyces pIJ101, pIJ364, pIJ702 oder pIJ361, in Bacillus pUB110, pC194 oder pBD214, in Corynebacterium pSA77 oder pAJ667, in Pilzen pALS1, pIL2 oder pBB116, in Hefen 2alphaM, pAG-1, YEp6, YEp13 oder pEMBLYe23 oder in Pflanzen pLGV23, $pGHlac^{+}$, pBIN19, pAK2004 oder pDH51. Die genannten Plasmide stellen eine kleine Auswahl der möglichen Plasmide dar. Weitere Plasmide sind dem Fachmann wohl bekannt und können beispielsweise aus dem Buch Cloning Vectors (Eds. Pouwels P. H. et al. Elsevier, Amsterdam-New York-Oxford, 1985, ISBN 0 444 904018) entnommen werden.

Vorteilhafterweise enthält das Nukleinsäurekonstrukt zur Expression der weiteren enthaltenen Gene zusätzlich noch 3'- und/oder 5'-terminale regulatorische Sequenzen zur Steigerung der Expression, die je nach ausgewähltem Wirtorganismus und Gen oder Gene für eine optimale Expression ausgewählt werden.

Diese regulatorischen Sequenzen sollen die gezielte Expression der Gene und der Proteinexpression ermöglichen. Dies kann beispielsweise je nach Wirtsorganismus bedeuten, dass das Gen erst nach Induktion exprimiert oder überexprimiert wird, oder dass es sofort exprimiert und/oder überexprimiert wird.

Die regulatorischen Sequenzen bzw. Faktoren können dabei vorzugsweise die Genexpression der eingeführten Gene positiv beeinflussen und dadurch erhöhen. So kann eine Verstärkung der regulatorischen Elemente vorteilhafterweise auf der Transkriptionsebene erfolgen, indem starke Transkriptionssignale wie Promotoren und/oder "Enhancer" verwendet werden. Daneben ist aber auch eine Verstärkung der Translation möglich, indem beispielsweise die Stabilität der mRNA verbessert wird.

In einer weiteren Ausgestaltungsform des Vektors kann der das Nukleinsäurekonstrukt oder die Nukleinsäure enthaltende Vektor auch vorteilhafterweise in Form einer linearen DNA in die Mikroorganismen eingeführt werden und über heterologe oder homologe Rekombination in das Genom des Wirtsorganismus integriert werden. Diese lineare DNA kann aus einem linearisierten Vektor wie einem Plasmid oder nur aus dem Nukleinsäurekonstrukt oder der hierin beschriebenen Nukleinsäure bestehen.

Für eine optimale Expression heterologer Gene in Organismen ist es vorteilhaft die Nukleinsäuresequenzen entsprechend des im Organismus verwendeten spezifischen "codon usage" zu verändern. Der "codon usage" lässt sich anhand von Computerauswertungen anderer, bekannter Gene des betreffenden Organismus leicht ermitteln.

Die Herstellung einer hierin beschriebenen Expressionskassette erfolgt durch Fusion eines geeigneten Promotors mit einer geeigneten kodierenden Nukleotidsequenz sowie einem Terminator- oder Polyadenylierungssignal. Dazu verwendet man gängige Rekombinations- und Klonierungstechniken, wie sie beispielsweise in T. Maniatis, E.F. Fritsch und J. Sambrook, Molecular Cloning: A Laboratory Manual, Cold Spring Harbor Laboratory, Cold Spring Harbor, NY (1989) sowie in T.J. Silhavy, M.L. Berman und L.W. Enquist, Experiments with Gene Fusions, Cold Spring Harbor

Laboratory, Cold Spring Harbor, NY (1984) und in Ausubel, F.M. et al., Current Protocols in Molecular Biology, Greene Publishing Assoc. and Wiley Interscience (1987) beschrieben sind.

Das rekombinante Nukleinsäurekonstrukt bzw. Genkonstrukt wird zur Expression in einem geeigneten Wirtsorganismus vorteilhafterweise in einen wirtsspezifischen Vektor insertiert, der eine optimale Expression der Gene im Wirt ermöglicht. Vektoren sind dem Fachmann wohl bekannt und können beispielsweise aus "Cloning Vectors" (Pouwels P. H. et al., Hrsg, Elsevier, Amsterdam-New York-Oxford, 1985) entnommen werden.

4.4 Brauchbare Wirte

Mit Hilfe der hierin beschriebenen Vektoren sind rekombinante Mikroorganismen herstellbar, welche beispielsweise mit wenigstens einem hierin beschriebenen Vektor transformiert sind und zur Produktion der verwendeten Polypeptide oder zur Durchführung der erfindungsgemäßen enzymatischen Reaktion eingesetzt werden können.

Vorteilhafterweise werden die oben beschriebenen rekombinanten Konstrukte in ein geeignetes Wirtssystem eingebracht und exprimiert. Dabei werden vorzugsweise dem Fachmann bekannte geläufige Klonierungs- und Transfektionsmethoden, wie beispielsweise Co-Präzipitation, Protoplastenfusion, Elektroporation, retrovirale Transfektion und dergleichen, verwendet, um die genannten Nukleinsäuren im jeweiligen Expressionssystem zur Expression zu bringen. Geeignete Systeme werden beispielsweise in Current Protocols in Molecular Biology, F. Ausubel et al., Hrsg., Wiley Interscience, New York 1997, oder Sambrook et al. Molecular Cloning: A Laboratory Manual. 2. Aufl., Cold Spring Harbor Laboratory, Cold Spring Harbor Laboratory Press, Cold Spring Harbor, NY, 1989 beschrieben.

Es sind auch homolog rekombinierte Mikroorganismen herstellbar. Dazu wird ein Vektor hergestellt, der zumindest einen Abschnitt eines hierin beschriebenen Gens oder einer kodierenden Sequenz enthält, worin gegebenenfalls wenigstens eine Aminosäure-Deletion, -Addition oder -Substitution eingebracht worden ist, um die Sequenz zu verändern, z.B. funktionell zu disrumpieren ("Knockout"-Vektor). Die eingebrachte Sequenz kann z.B. auch ein Homologes aus einem verwandten Mikroorganismus sein oder aus einer Säugetier-, Hefe- oder Insektenquelle abgeleitet sein. Der zur homologen Rekombination verwendete Vektor kann alternativ derart ausgestaltet sein, daß das endogene Gen bei homologer Rekombination mutiert oder anderweitig verändert ist, jedoch noch das funktionelle Protein kodiert (z.B. kann der stromaufwärts gelegene regulatorische Bereich derart verändert sein, dass dadurch die Expression des endogenen Proteins verändert wird). Der veränderte Abschnitt des Gens ist im homologen Rekombinationsvektor. Die Konstruktion geeigneter Vektoren zur homologen Rekombination ist z.B. beschrieben in Thomas, K.R. und Capecchi, M.R. (1987) Cell 51:503.

Als rekombinante Wirtsorganismen für die hierin beschriebenen Nukleinsäure oder dem Nukleinsäurekonstrukt kommen prinzipiell alle prokaryontischen oder eukaryontischen Organismen in Frage. Vorteilhafterweise werden als Wirtsorganismen Mikroorganismen wie Bakterien, Pilze oder Hefen verwendet. Vorteilhaft werden gram-positive oder gram-negative Bakterien, bevorzugt Bakterien der Familien Enterobacteriaceae, Pseudomonadaceae, Rhizobiaceae, Streptomycetaceae, Bacillaceae oder Nocardiaceae, besonders bevorzugt Bakterien der Gattungen Escherichia, Pseudomonas, Streptomyces, Nocardia, Burkholderia, Salmonella, Agrobacterium, Bacillus oder Rhodococcus verwendet. Ganz besonders bevorzugt ist die Gattung und Art Escherichia coli. Weitere vorteilhafte Bakterien sind darüber hinaus in der Gruppe der alpha-Proteobacterien, beta-Proteobacterien oder gamma-Proteobacterien zu finden.

Der Wirtsorganismus oder die Wirtsorganismen enthalten dabei vorzugsweise mindestens eine der beschriebenen Nukleinsäuresequenzen, Nukleinsäurekonstrukte oder Vektoren, die für ein ADH Enzym kodieren.

Die im erfindungsgemäßen Verfahren verwendeten Organismen werden je nach Wirtsorganismus in dem Fachmann bekannter Weise angezogen bzw. gezüchtet. Mikroorganismen werden in der Regel in einem flüssigen Medium, das eine Kohlenstoffquelle meist in Form von Zuckern, eine Stickstoffquelle meist in Form von organischen Stickstoffquellen wie Hefeextrakt oder Salzen wie Ammoniumsulfat, Spurenelemente wie Eisen-, Mangan-, Magnesiumsalze und gegebenenfalls Vitamine enthält, bei Temperaturen zwischen 0°C und 100 °C, bevorzugt zwischen 10 °C bis 60 °C unter Sauerstoffbegasung angezogen. Dabei kann der pH der Nährflüssigkeit auf einen festen Wert gehalten werden, das heißt während der Anzucht reguliert werden oder nicht. Die Anzucht kann "batch"-weise, "semi batch"-weise oder kontinuierlich erfolgen. Nährstoffe können zu Beginn der Fermentation vorgelegt oder semikontinuierlich oder kontinuierlich nachgefüttert werden.

Das umzusetzende Keton kann direkt zur Anzucht gegeben werden oder vorteilhaft nach Anzucht.

Die Enzyme können entweder aus den Organismen isoliert werden oder als Rohextrakt für die Reaktion verwendet werden.

Die Wirtsorganismen enthalten z.B.1 U/I Enzymaktivität, wie z.B ADH-Aktivität, bevorzugt 100 U/I, besonders bevorzugt mehr als 1000 U/I.

4.5 Rekombinante Herstellung von Enzymen:

Die eingesetzten Enzyme sind auch durch rekombinante Herstellung zugänglich, wobei man einen dieses

Enzym produzierenden Mikroorganismus kultiviert, gegebenenfalls die Expression der Polypeptide induziert und diese aus der Kultur isoliert. Die Polypeptide können so auch in großtechnischem Maßstab produziert werden, falls dies erwünscht ist.

Der rekombinante Mikroorganismus kann nach bekannten Verfahren kultiviert und fermentiert werden. Bakterien können beispielsweise in TB- oder LB-Medium und bei einer Temperatur von 20 bis 40°C und einem pH-Wert von 6 bis 9 vermehrt werden. Im Einzelnen werden geeignete Kultivierungsbedingungen beispielsweise in T. Maniatis, E.F. Fritsch and J. Sambrook, Molecular Cloning: A Laboratory Manual, Cold Spring Harbor Laboratory, Cold Spring Harbor, NY (1989) beschrieben.

Die Zellen werden dann, falls die Polypeptide nicht in das Kulturmedium sezerniert werden, aufgeschlossen und das Produkt nach bekannten Proteinisolierungsverfahren aus dem Lysat gewonnen. Die Zellen können wahlweise durch hochfrequenten Ultraschall, durch hohen Druck, wie z.B. in einer French-Druckzelle, durch Osmolyse, durch Einwirkung von Detergenzien, lytischen Enzymen oder organischen Lösungsmitteln, durch Homogenisatoren oder durch Kombination mehrerer der aufgeführten Verfahren aufgeschlossen werden.

Eine Aufreinigung der Polypeptide kann mit bekannten, chromatographischen Verfahren erzielt werden, wie Molekularsieb-Chromatographie (Gelfiltration), wie Q-Sepharose-Chromatographie, Ionenaustausch-Chromatographie und hydrophobe Chromatographie, sowie mit anderen üblichen Verfahren wie Ultrafiltration, Kristallisation, Aussalzen, Dialyse und nativer Gelelektrophorese. Geeignete Verfahren werden beispielsweise in Cooper, F. G., Biochemische Arbeitsmethoden, Verlag Walter de Gruyter, Berlin, New York oder in Scopes, R., Protein Purification, Springer Verlag, New York, Heidelberg, Berlin beschrieben.

Vorteilhaft kann es sein, zur Isolierung des rekombinanten Proteins Vektorsysteme oder Oligonukleotide zu verwenden, die die cDNA um bestimmte Nukleotidsequenzen verlängern und damit für veränderte Polypeptide oder Fusionsproteine kodieren, die z.B. einer einfacheren Reinigung dienen. Derartige geeignete Modifikationen sind beispielsweise als Anker fungierende sogenannte "Tags", wie z.B. die als Hexa-Histidin-Anker bekannte Modifikation oder Epitope, die als Antigene von Antikörpern erkannt werden können (beschrieben zum Beispiel in Harlow, E. and Lane, D., 1988, Antibodies: A Laboratory Manual. Cold Spring Harbor (N.Y.) Press). Diese Anker können zur Anheftung der Proteine an einen festen Träger, wie z.B. einer Polymermatrix, dienen, die beispielsweise in einer Chromatographiesäule eingefüllt sein kann, oder an einer Mikrotiterplatte oder an einem sonstigen Träger verwendet werden kann.

Gleichzeitig können diese Anker auch zur Erkennung der Proteine verwendet werden. Zur Erkennung der Proteine können außerdem übliche Marker, wie Fluoreszenzfarbstoffe, Enzymmarker, die nach Reaktion mit einem Substrat ein detektierbares Reaktionsprodukt bilden, oder radioaktive Marker, allein oder in Kombination mit den Ankern zur Derivatisierung der Proteine verwendet werden.

4.6 Durchführung des erfindungsgemäßen Verfahrensschritts b) zur Herstellung von optisch aktiven Alkoholen

Die verwendeten Enzyme, können im erfindungsgemäßen Verfahrensschritt als freies oder immobilisiertes Enzym verwendet werden.

Der erfindungsgemäße Verfahrensschritt wird vorteilhaft bei einer Temperatur zwischen 0 °C bis 60 °C, bevorzugt zwischen 10 °C bis 40 °C, besonders bevorzugt zwischen 15 °C bis 35 °C durchgeführt.

Der pH-Wert während des erfindungsgemäßen Verfahrensschritts wird vorteilhaft zwischen pH 4 und 12, bevorzugt zwischen pH 4,5 und 9, besonders bevorzugt zwischen pH 5 und 8 gehalten.

Für das erfindungsgemäße Verfahren können wachsende Zellen verwendet werden, die die erfindungsgemäßen Nukleinsäuren, Nukleinsäurekonstrukte oder Vektoren enthalten. Auch ruhende oder aufgeschlossene Zellen können verwendet werden. Unter aufgeschlossenen Zellen sind beispielsweise Zellen zu verstehen, die über eine Behandlung mit beispielsweise Lösungsmitteln durchlässig gemacht worden sind, oder Zellen die über eine Enzymbehandlung, über eine mechanische Behandlung (z.B. French Press oder Ultraschall) oder über eine sonstige Methode aufgebrochen wurden. Die so erhaltenen Rohextrakte sind für das erfindungsgemäße Verfahren geeignet. Auch gereinigte oder angereinigte Enzyme können für das Verfahren verwendet werden. Ebenfalls geeignet sind immobilisierte Mikroorganismen oder Enzyme.

Werden für das erfindungsgemäße Verfahren freie Organismen oder Enzyme verwendet, so werden diese vor der Extraktion zweckmäßigerweise abgetrennt, beispielsweise über eine Filtration oder Zentrifugation.

Wird ein zweiphasiges (wässrig/organisches) Reaktionsmedium verwendet, so wird dadurch die Produktisolierung erleichtert, da sich das Wertprodukt bevorzugt in der organischen Phase lösen kann. Beispielsweise verwendet man insbesondere ein mit Wasser im wesentlichen nicht mischbares Lösungsmittel, wie z.B. einen Äther, um das zweiphasige System auszubilden.

Wird dagegen ein einphasiges Reaktionsmedium im enzymatischen Verfahrenschritt verwendet, so lässt sich das hergestellte Produkt aus der wässrigen Reaktionslösung über Extraktion oder Destillation oder vorteilhaft über Extraktion und Destillation gewinnen. Die Extraktion kann zur Erhöhung der Ausbeute mehrfach wiederholt werden. Beispiele für geeignete Extraktionsmittel sind Lösungsmittel, wie Toluol, Methylenchlorid, Butylacetat, Diisopropylether,

Benzol, MTBE oder Essigester, ohne darauf beschränkt zu sein.

Nach Einengen der organischen Phase können die Produkte in der Regel in guten chemischen Reinheiten, das heißt in mehr als 80 %, 90 %, 95 % oder 99 % chemischer Reinheit, gewonnen werden. Nach Extraktion kann die organische Phase mit dem Produkt aber auch nur zum Teil eingeengt werden und das Produkt auskristallisiert werden. Dazu wird die Lösung vorteilhaft auf eine Temperatur von 0 °C bis 10 °C abgekühlt. Die Kristallisation kann auch direkt aus der organischen Lösung oder aus einer wässrigen Lösung erfolgen. Das auskristallisierte Produkt kann nochmals im gleichen oder in einem anderen Lösungsmittel zur erneuten Kristallisation aufgenommen werden und nochmals kristallisiert werden. Durch die anschließende vorteilhafte mindestens einmalig durchgeführte Kristallisation kann die Enantiomerenreinheit des Produktes falls erforderlich weiter gesteigert werden.

Bei den genannten Aufarbeitungsarten lässt sich das Produkt des erfindungsgemäßen Verfahrens in Ausbeuten von 60 bis 100 %, bevorzugt von 80 bis 100 %, besonders bevorzugt von 90 bis 100 %, bezogen auf das für die Reaktion eingesetzte Substrat, isolieren. Das isolierte Produkt zeichnet sich durch eine hohe chemische Reinheit von > 90 %, bevorzugt > 95 % besonders bevorzugt von > 98 % aus. Weiterhin haben die Produkt eine hohe Enantiomerenreinheit, die vorteilhaft falls erforderlich durch die Kristallisation weiter gesteigert werden kann.

Das erfindungsgemäße Verfahren kann batchweise, semi-batchweise oder kontinuierlich betrieben werden.

Die Durchführung des Verfahrens kann vorteilhafterweise in Bioreaktoren erfolgen, wie z.B. beschrieben in Biotechnology, Band 3, 2. Auflage, Rehm et al Hrsg., (1993) insbesondere Kapitel II.

Die obige Beschreibung und die nachstehenden Beispiele dienen nur der Verdeutlichung der Erfindung. Die für den Fachmann offensichtlichen, zahlreich möglichen Abwandlungen sind ebenfalls möglich.

## Experimenteller Teil:

**Beispiel 1:** Synthese von HCAP, **2** (in Ethylacetat):

In einem 6000 ml Miniplant-Reaktor mit Impeller Rührer, Stromstörer, Thermometer und Tropftrichter werden 435,68g (3,20 Mol) 3-Hydroxyacetophenon in 410,11g (12,80 Mol) Methanol und 1200 ml Ethylacetat vorgelegt. Zu dieser Lösung werden bei 20 - 30°C unter Kühlung 691,05g (5,12 Mol) Sulfurylchlorid innerhalb 2h zugetropft. Nach dem Zutropfen wird der Ansatz eine Stunde bei Raumtemperatur weiter gerührt. Danach wird der Ansatz bei Raumtemperatur mit 1600 ml $H_2O$ hydrolysiert und die resultierende zweiphasige Mischung getrennt. Die wässrige Phase wird noch einmal mit 800 ml Ethylacetat extrahiert. Aus den vereinigten organischen Phasen werden das Methanol und der Ethylacetat über eine Destillationsbrücke abdestilliert. Gleichzeitig werden 1880 ml Isopropanol in den Destillationssumpf zugetropft. Man erhält 2462,5g einer 17,3% isopropanolischen Lösung des Wertproduktes was einem Gehalt von 426 g (2,51 Mol) entspricht. Die Ausbeute beträgt somit 78%.

**Beispiel 2:** Synthese von HCAP, **2** (in Dichlormethan):

In einem 2000 ml Miniplant-Reaktor mit Impeller Rührer, Stromstörer, Thermometer und Tropftrichter werden 204,23g (1,50 Mol) 3-Hydroxyacetophenon in 192,24g (6,00 Mol) Methanol und 1050ml $CH_2Cl_2$ vorgelegt. Zu dieser Lösung werden bei 20 - 30°C unter Kühlung 283,44g (2,10 Mol) Sulfurylchlorid innerhalb von 2 Stunden zugetropft. Nach dem Zutropfen wird der Ansatz eine Stunde bei Raumtemperatur weiter gerührt.

Danach wird der Ansatz bei Raumtemperatur mit 400 ml $H_2O$ hydrolysiert und die resultierende zweiphasige Mischung getrennt. Nach der Phasentrennung werden aus der organischen Phase das Methanol und das $CH_2Cl_2$ über eine Destillationsbrücke bei Normaldruck abdestilliert. Gleichzeitig werden mit der gleichen Geschwindigkeit 880ml Isopropanol zugetropft. Man erhält 837,78g einer 25,7% isopropanolischen Lösung des Wertproduktes was einem Gehalt von 215 g (1,26 Mol) entspricht. Die Ausbeute beträgt somit 84%.

**Beispiel 3:** Synthese von HCPE, **3:**

Ein wie in Beispiel 1 oder 2 hergestelltes Keton **2** wird biokatalytisch zu ***R*-3** reduziert. Dazu werden in einem geeigneten Rührbehälter in 1,44 L wässrigen Kaliumphosphatpuffer (50mM, pH 7) 1mM $MgCl_2$, 0,02 mM Nicotinamidadenindinucleotid (NAD) und 282g *i*-Propanol gelöst, das auch als Opferalkohol zur Cofaktorregenerierung dient. Als Katalysator werden Zellen rekombinanter *Escherichia coli* (entsprechend 3,75 g Biotrockenmasse) verwendet, die eine stereoselektive Dehydrogenase (E.C. 1.1.1.1) überproduzieren. Die Herstellung eines geeigneten Biokatalysators ist in WO 2005/108590, Beispiel 1-3 beschrieben, worauf hiermit ausdrücklich Bezug genommen wird. Die wässrige Phase wird mit 1,3 kg M*t*BE überschichtet. 292,8 g **2** (als isopropanolische Lösung) werden der Reaktionsmischung zudosiert. Im Reaktionsansatz sollte die Konzentration von **2** nicht über 50mM steigen. Die Umsetzung kann mittels achiraler oder chiraler Chromatographie verfolgt werden.

Nach der Reaktion trennen sich organische und wässrige Phase auf Grund ihrer unterschiedlichen spezifischen

Gewichte. Das Wertprodukt ***R*-3** befindet sich vornehmlich in der M*t*BE-Phase.

**Beispiel 4:** Synthese von Phenylephrin, **4:**

15 g (86,9 mmol) der Verbindung **R-3** werden in 85 ml THF gelöst und im Druckautoklaven bei 90°C mit 13,5 g (435 mmol) Methylamin zur Reaktion gebracht. Man lässt so lange reagieren, bis das Edukt vollständig umgesetzt ist (ca. 5 Stunden). Anschließend wird auf Raumtemperatur abgekühlt und die erhaltenen Suspension eingeengt. Durch Zugabe von 100g Wasser wird die freie Base des Wertproduktes ausgefällt und isoliert. Man erhält 12,85 g (76,8 mmol, 88%) Phenylephrin freie Base.

## Patentansprüche

**1.** Verfahren zur Herstellung von substituierten, optisch aktiven Alkoholen der Formel IV

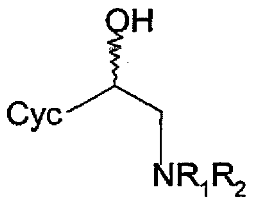

(IV)

worin

Cyc für einen ein- oder mehrkernigen, gesättigten oder ungesättigten, carbocyclischen oder heterocyclischen, gegebenenfalls ein- oder mehrfach substituierten Ring steht, der wenigstens eine freie Hydroxylgruppe trägt, und $R_1$ und $R_2$ unabhängig voneinander für H oder einen gegebenenfalls ein- oder mehrfach substituierten Alkylrest stehen;

oder von Salzen dieser Verbindung; jeweils in stereoisomerenreiner Form oder als Gemisch von Stereoisomeren,

wobei man

a) ein Keton der Formel I

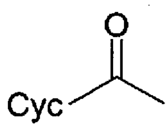

(I)

worin Cyc die oben angegebenen Bedeutungen besitzt,
in Gegenwart eines aliphatischen Alkohols mit einem Halogenierungsmittel zu einer halogenierten Verbindung der Formel II

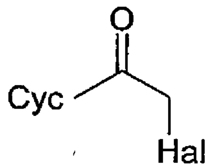

(II)

umsetzt, worin Cyc die oben angegebenen Bedeutungen besitzt und Hal für ein Halogenatom steht;
b) die so erhaltene Verbindung der Formel II enzymatisch unter Verwendung eines Enzyms, ausgewählt unter Alkoholdehydrogenasen (ADH) (E.C. 1.1.1.1), zum Alkohol der Formel III

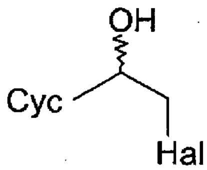

(III)

reduziert, worin Cyc und Hal die oben angegebenen Bedeutungen besitzen; und
c) den so erhaltenen Alkohol der Formel III mit einem Amin der Formel $HNR_1R_2$, worin $R_1$ und $R_2$ die oben angegebenen Bedeutungen besitzen; zur Verbindung der Formel IV umsetzt.

2. Verfahren nach Anspruch 1, wobei die Umsetzung der Stufe a) in Gegenwart von 1 bis 10 Moläquivalenten Alkohol je Mol Alkanon der Formel I erfolgt.

3. Verfahren nach einem der vorhergehenden Ansprüche, wobei die chemische Umsetzung von Stufe c) in Lösung in einem offenkettigen oder zyklischen Ether erfolgt.

4. Verfahren nach Anspruch 1, wobei die Alkoholdehydrogenase ausgewählt ist unter Enzymen aus Mikroorganismen der Gattung *Aromatoleum.*

5. Verfahren nach einem der vorhergehenden Ansprüche, wobei das Enzym für Stufe b) ausgewählt ist unter Enzymen, welche eine Polypeptidsequenz besitzen, die ausgewählt ist unter

(i) SEQ ID NO: 2 ist, oder
(ii) Sequenzen worin bis zu 25% der Aminosäurereste gegenüber SEQ ID NO: 2 durch Addition, Deletion, Insertion, Substitution, Inversion oder einer Kombination davon verändert sind, und / oder die noch mindestens 50% der enzymatischen Aktivität von SEQ ID NO:2 aufweiset.

6. Verfahren nach einem der vorhergehenden Ansprüche, wobei man die Umsetzung von Stufe b) unter Zugabe von Reduktionsäquivalenten durchführt und gegebenenfalls die bei der Umsetzung verbrauchten Reduktionsäquivalente regeneriert.

7. Verfahren nach Anspruch 6, wobei man die Regeneration enzymatisch, elektrochemisch oder elektroenzymatisch durchführt.

8. Verfahren nach Anspruch 7, wobei die Regeneration enzymatisch erfolgt und das regenerierende Enzym ausgewählt

ist unter ADH und von ADH verschiedenen Dehydrogenasen.

**9.** Verfahren nach einem der vorhergehenden Ansprüche, wobei die Umsetzung von Stufe b) in Gegenwart eines Mikroorganismus, der die ADH natürlich oder rekombinant exprimiert, oder in Gegenwart einer davon abgeleiteten, die ADH enthaltenden Fraktion, oder in Gegenwart eines davon abgeleiteten, die ADH enthaltenden Extraktes erfolgt.

**10.** Verfahren nach einem der vorhergehenden Ansprüche, wobei die Umsetzung von Stufe b) in Gegenwart eines ADH produzierenden Mikroorganismus, der ausgewählt ist unter Bakterien der Familien Enterobacteriaceae, Pseudomonadaceae, Rhizobiaceae, Lactobacillaceae, Streptomycetaceae, Rhodococcaceae, Rhodocyclaceae und Nocardiaceae, oder in Gegenwart einer oder eines davon abgeleiteten, ADH-haltigen Fraktion oder Extraktes erfolgt.

**11.** Verfahren nach Anspruch 10, wobei der Mikroorganismus ein rekombinanter Mikroorganismus ist, der mit einem Nukleinsäurekonstrukt transformiert ist, welche für eine Alkoholdehydrogenase gemäß der Definition in einem der Ansprüche 4 bis 6 kodiert.

**12.** Verfahren nach einem der vorhergehenden Ansprüche, wobei man Stufe b) in einem zweiphasigen flüssigen Reaktionsmedium durchführt.

**13.** Verfahren nach Anspruch 12, wobei ein wässrig-organisches Reaktionsmedium verwendet wird, wobei sowohl das Edukt der Formel II als auch das Produkt der Formel III in der organischen Phase besser löslich sind als in der wässrigen Phase.

**14.** Verwendung einer Alkoholdehydrogenase gemäß der Definition in einem der Ansprüche 1 bis 6 oder eines dieses Enzym produzierenden Mikroorganismus zur Herstellung einer Verbindung der Formel IV.

## Claims

**1.** A method of production of substituted, optically active alcohols of formula IV

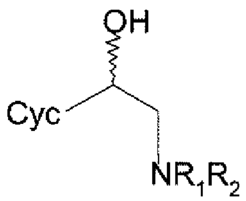

(IV)

in which

Cyc stands for a mono- or polynuclear, saturated or unsaturated, carbocyclic or heterocyclic, optionally singly or multiply substituted ring, which has at least one free hydroxyl group, and
$R_1$ and $R_2$ independently of one another stand for H or an optionally singly or multiply substituted alkyl residue; or of salts of this compound; in each case in stereoisomerically pure form or as a mixture of stereoisomers,

wherein

a) a ketone of formula I

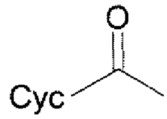

(I)

in which Cyc has the meanings stated above,
is reacted in the presence of an aliphatic alcohol with a halogenating agent to a halogenated compound of formula II

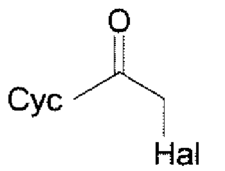

(II)

in which Cyc has the meanings stated above and Hal stands for a halogen atom;

b) the resultant compound of formula II is reduced enzymatically using an enzyme, selected from alcohol dehydrogenases (ADH) (E.C. 1.1.1.1), to the alcohol of formula III

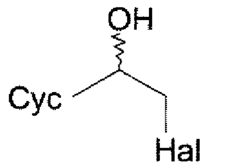

(III)

in which Cyc and Hal have the meanings stated above; and

c) the resultant alcohol of formula III is reacted with an amine of formula $HNR_1R_2$, in which $R_1$ and $R_2$ have the meanings stated above, to the compound of formula IV.

**2.** The method according to claim 1, wherein the reaction in stage a) takes place in the presence of 1 to 10 molar equivalents of alcohol per mol of alkanone of formula I.

**3.** The method according to one of the preceding claims, wherein the chemical reaction in stage c) takes place in solution in an open-chain or cyclic ether.

**4.** The method according to claim 1, wherein the alcohol dehydrogenase is selected from enzymes from microorganisms of the genus *Aromatoleum.*

**5.** The method according to one of the preceding claims, wherein the enzyme for stage b) is selected from enzymes that have a polypeptide sequence that is selected from

(i) SEQ ID NO: 2, or

(ii) sequences in which up to 25% of the amino acid residues are altered relative to SEQ ID NO: 2 by addition, deletion, insertion, substitution, inversion or a combination thereof, and/or that still have at least 50% of the enzymatic activity of SEQ ID NO:2.

**6.** The method according to one of the preceding claims, wherein the reaction in stage b) is carried out with addition of reduction equivalents and optionally the reduction equivalents consumed during the reaction are regenerated.

**7.** The method according to claim 6, wherein the regeneration is carried out enzymatically, electrochemically or electro-enzymatically.

**8.** The method according to claim 7, wherein the regeneration takes place enzymatically and the regenerating enzyme is selected from ADH and dehydrogenases different from ADH.

**9.** The method according to one of the preceding claims, wherein the reaction in stage b) takes place in the presence of a microorganism, which expresses the ADH naturally or recombinantly, or in the presence of a fraction derived therefrom, containing the ADH, or in the presence of an extract derived therefrom, containing the ADH.

**10.** The method according to one of the preceding claims, wherein the reaction in stage b) takes place in the presence of an ADH-producing microorganism, which is selected from bacteria of the families Enterobacteriaceae, Pseudomonadaceae, Rhizobiaceae, Lactobacillaceae, Streptomycetaceae, Rhodococcaceae, Rhodocyclaceae and Nocardiaceae, or in the presence of an ADH-containing fraction or extract derived therefrom.

**11.** The method according to claim 10, wherein the microorganism is a recombinant microorganism, which is transformed

with a nucleic acid construct that codes for an alcohol dehydrogenase according to the definition in one of claims 4 to 6.

**12.** The method according to one of the preceding claims, wherein stage b) is carried out in a two-phase liquid reaction medium.

**13.** The method according to claim 12, wherein an aqueous-organic reaction medium is used, and both the educt of formula II and the product of formula III are more soluble in the organic phase than in the aqueous phase.

**14.** Use of an alcohol dehydrogenase according to the definition in one of claims 1 to 6 or a microorganism producing this enzyme for the production of a compound of formula IV.

**Revendications**

**1.** Procédé pour la préparation d'alcools substitués, optiquement actifs de formule IV

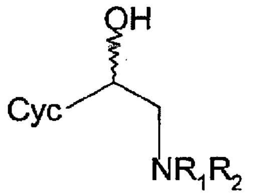

(IV)

dans laquelle

Cyc représente un cycle à un ou plusieurs noyaux, saturé ou insaturé, carbocyclique ou hétérocyclique, le cas échéant monosubstitué ou polysubstitué, qui porte au moins un groupe hydroxyle libre et
$R_1$ et $R_2$ représentent, indépendamment l'un de l'autre, H ou un radical alkyle, le cas échéant monosubstitué ou polysubstitué
ou de sels de ce composé ; à chaque fois sous forme pure sur le plan des stéréo-isomères ou sous forme de mélange de stéréo-isomères, dans lequel

a) une cétone de formule I

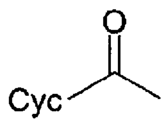

(I)

dans laquelle Cyc présente les significations indiquées ci-dessus, est transformée en présence d'un alcool aliphatique avec un agent d'halogénation en un composé halogéné de formule II

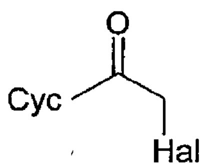

(II)

dans laquelle Cyc présente les significations indiquées ci-dessus et Hal représente un atome d'halogène;

b) le composé ainsi obtenu de formule II est réduit enzymatiquement, en utilisant une enzyme choisie parmi les alcool-déshydrogénases (ADH) (E.C. 1.1.1.1), en alcool de formule III

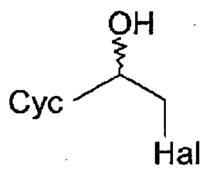

(III)

dans laquelle Cyc et Hal présentent les significations indiquées ci-dessus ; et
c) l'alcool de formule III ainsi obtenu est transformé avec une amine de formule $HNR_1R_2$, dans laquelle $R_1$ et $R_2$ présentent les significations indiquées ci-dessus, en composé de formule IV.

**2.** Procédé selon la revendication 1, la transformation de l'étape a) ayant lieu en présence de 1 à 10 équivalents en mole par mole d'alcanone de formule I.

**3.** Procédé selon l'une quelconque des revendications précédentes, la transformation chimique de l'étape c) ayant lieu en solution dans un éther à chaîne ouverte ou cyclique.

**4.** Procédé selon la revendication 1, l'alcool-déshydrogénase étant choisie parmi les enzymes provenant de micro-organismes du genre Aromatoleum.

**5.** Procédé selon l'une quelconque des revendications précédentes, l'enzyme pour l'étape b) étant choisie parmi des enzymes qui présentent une séquence polypeptidique qui est choisie parmi

(i) la séquence SEQ ID NO : 2, ou
(ii) des séquences dans lesquelles jusqu'à 25% des restes d'acides aminés sont modifiés par rapport à la séquence SEQ ID NO : 2 par addition, délétion, insertion, substitution, inversion ou une combinaison de celles-ci et/ou qui présentent encore au moins 50% de l'activité enzymatique de la SEQ ID NO : 2.

**6.** Procédé selon l'une quelconque des revendications précédentes, la transformation de l'étape b) étant réalisée avec addition d'équivalents de réduction et, le cas échéant, les équivalents de réduction consommés lors de la transformation étant régénérés.

**7.** Procédé selon la revendication 6, la régénération enzymatique étant réalisée par voie enzymatique, électrochimique ou électro-enzymatique.

**8.** Procédé selon la revendication 7, la régénération ayant lieu par voie enzymatique et l'enzyme de régénération étant choisie parmi l'ADH et des déshydrogénases différentes de l'ADH.

**9.** Procédé selon l'une quelconque des revendications précédentes, la transformation de l'étape b) ayant lieu en présence d'un micro-organisme qui exprime l'ADH de manière naturelle ou recombinante ou en présence d'une fraction dérivée de celui-ci, contenant l'ADH ou en présence d'un extrait dérivé de celui-ci, contenant l'ADH.

**10.** Procédé selon l'une quelconque des revendications précédentes, la transformation de l'étape b) ayant lieu en présence d'un micro-organisme produisant de l'ADH, qui est choisi parmi les bactéries des familles des Enterobacteriaceae, des Pseudomonadaceae, des Rhizobiaceae, des Lactobacillaceae, des Streptomycetaceae, des Rhodococcaceae, des Rhodocyclaceae et des Nocardiaceae ou en présence d'une fraction ou d'un extrait dérivé(e) de celui-ci, contenant l'ADH.

**11.** Procédé selon la revendication 10, le micro-organisme étant un micro-organisme recombinant, qui est transformé avec un élément d'acide nucléique qui code pour une alcool-déshydrogénase selon la définition dans l'une quelconque des revendications 4 à 6.

**12.** Procédé selon l'une quelconque des revendications précédentes, l'étape b) étant réalisée dans un milieu de réaction liquide à deux phases.

**13.** Procédé selon la revendication 12, un milieu de réaction aqueux-alcoolique étant utilisé, tant le produit de départ de formule II que le produit de formule III étant mieux solubles dans la phase organique que dans la phase aqueuse.

**14.** Utilisation d'une alcool-déshydrogénase selon la définition dans l'une quelconque des revendications 1 à 6 ou d'un micro-organisme produisant cette enzyme pour la production d'un composé de formule IV.

SEQ ID NO:1 Nukleinsäuresäuresequenz der Phenylethanol-Dehydrogenase aus *Azoarcus* sp EbN1 (Genbank ID 25956124, Region:25073 bis 25822)

```
  1 atgacgcaaa gactgaagga caagcttgca gtaattaccg gcggtgccaa cggcatcggg
 61 cgggcaattg cggagcgatt tgcggtcgaa ggtgccgaca tcgcaatcgc ggatctggtg
121 ccggccccgg aagccgaggc agcaatcagg aacctcggtc ggcgcgttct gaccgtgaag
181 tgcgatgtct cgcaacctgg cgacgtagaa gcattcggaa agcaggtcat ctccacgttt
241 ggtcgctgcg acatcctcgt caacaacgcg ggaatttacc cgctgattcc ttttgacgag
301 ctgacctttg aacagtggaa gaaaacattc gagatcaacg tcgattcagg ttttcttatg
361 gcgaaggctt ttgtccccgg gatgaagagg aacgggtggg gacgcatcat caacctgact
421 tcgacgacat attggctaaa gatcgaggcg tatacccatt acatcagcac gaaagcggca
481 aacataggct ttacccgcgc ccttgcctcg gacctgggga aggacggaat cactgttaac
541 gccatcgcgc cgagccttgt ccgcacggca acaaccgaag cttctgcatt gtccgcgatg
601 ttcgacgtgc tgccaaacat gcttcaggcg attccgcgtc ttcaggtgcc cctggatctg
661 acgggcgcag ctgcgttcct ggcttccgat gacgccagtt ttattacagg ccagacgctc
721 gcggttgatg gcggtatggt gagacactga
```

SEQ ID NO:2 Aminosäuresequenz der Phenylethanol-Dehydrogenase aus *Azoarcus* sp EbN1 (Genbank protein ID CAD58337)

```
  1 MTQRLKDKLA VITGGANGIG RAIAERFAVE GADIAIADLV PAPEAEAAIR
 51 NLGRRVLTVK CDVSQPGDVE AFGKQVISTF GRCDILVNNA GIYPLIPFDE
101 LTFEQWKKTF EINVDSGFLM AKAFVPGMKR NGWGRIINLT STTYWLKIEA
151 YTHYISTKAA NIGFTRALAS DLGKDGITVN AIAPSLVRTA TTEASALSAM
201 FDVLPNMLQA IPRLQVPLDL TGAAAFLASD DASFITGQTL AVDGGMVRH
```

**Fig. 1**

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- WO 0043345 A
- WO 2005108590 A
- US 4310702 A
- US 5710341 A
- DE 2004022686
- EP 2005004872 A
- EP 06123814 A
- WO 2008055988 A3


### In der Beschreibung aufgeführte Nicht-Patentliteratur


- *Tetrahedron Letters,* 1989, vol. 30, 367-370
- *Chem. Pharm. Bull.,* 1995, vol. 43 (5), 738-747
- **ACHIWA et al.** *beschreiben in Tetrahedron Letters,* 1989, vol. 30, 367-370
- *Chem. Pharm. Bull.,* 1995, vol. 43 (5), 738-747
- *Tetrahedron Asymmetry,* 2007, vol. 18 (15), 1799-1803
- *Biotechnology Progress,* 2005, vol. 21, 1192
- *Biocatalysis and Biotransformation,* 2004, vol. 22, 89
- *Angew. Chem Int. Ed Engl.,* 2001, vol. 40, 169
- *Biotechnol Bioeng,* 2006, vol. 96, 18
- *Biotechnol Adv.,* 2007, vol. 25, 369
- *Angew.Chem Int.Ed Engl.,* 2008, vol. 47, 2275
- *Current Opinion in Biotechnology,* 2003, vol. 14, 421
- *Current Opinion in Biotechnology,* 2003, vol. 14, 583
- **D.P. WYMAN et al.** *J. Org. Chem.,* 1964, vol. 29, 1956-1960
- **D. MASILAMANI et al.** *J. Org. Chem.,* 1981, vol. 46, 4486-4489
- *Angew.Chem.Int.Ed.,* 2004, vol. 43, 788
- *Tetrahedron,* 2004, vol. 60, 633
- *Chiral catalysis - asymmetric hydrogenation supplement to Chemistry Today,* 2004, vol. 22, 26
- *Current Opinion in Chemical Biology,* 2004, vol. 8, 120
- *Organic Process Research & Development,* 2002, vol. 6, 558
- *Tetrahedron: Asymmetry,* 2003, vol. 14, 2659
- *Chiral catalysis - asymmetric hydrogenation supplement to Chemistry Today,* 2004, vol. 22, 43
- *Applied Microbiology and Biotechnology,* 1997, vol. 48, 699
- *Bioscience Biotechnology and Biochemistry,* 1998, vol. 62, 167
- *Methods Enzymol.,* 1987, vol. 136, 9
- *Ann. N. Y.Acad. Sci.,* 1984, vol. 434, 91
- *FEBS Journal,* 2005, vol. 272, 3816
- *Applied Microbiology and Biotechnology,* 2003, vol. 61, 133
- *Angew.Chem Int.Ed Engl.,* 2001, vol. 40, 169
- *Angewandte Chemie Int.Ed.Engl.,* 1999, vol. 29, 388
- *Comput Appl. Biosci.,* April 1989, vol. 5 (2), 151-1
- **NARANG, S.A.** *Tetrahedron,* 1983, vol. 39, 3
- **ITAKURA et al.** *Annu. Rev. Biochem.,* 1984, vol. 53, 323
- **ITAKURA et al.** *Science,* 1984, vol. 198, 1056
- **IKE et al.** *Nucleic Acids Res.,* 1983, vol. 11, 477
- **ARKIN ; YOURVAN.** *PNAS,* 1992, vol. 89, 7811-7815
- **DELGRAVE et al.** *Protein Engineering,* 1993, vol. 6 (3), 327-331
- **SAMBROOK et al.** Molecular Cloning: A laboratory manual. Cold Spring Harbor Laboratory Press, 1989
- **SAMBROOK, J. ; FRITSCH, E.F. ; MANIATIS, T.** Molecular Cloning: A Laboratory Manual. Cold Spring Harbor Laboratory Press, 1989
- **SAMBROOK et al.** Molecular Cloning. Cold Spring Harbor Laboratory, 1989
- Current Protocols in Molecular Biology. John Wiley & Sons, 1985
- Nucleic Acids Hybridization: A Practical Approach. IRL Press at Oxford University Press, 1985
- Essential Molecular Biology: A Practical Approach. IRL Press at Oxford University Press, 1991
- **AUSUBEL et al.** Current Protocols in Molecular Biology. John Wiley & Sons, 1989, 6.3.1-6.3.6
- **GOEDDEL.** Gene Expression Technology: Methods in Enzymology. Academic Press, 1990, vol. 185
- Cloning Vectors. Elsevier, 1985
- **T. MANIATIS ; E.F. ; J. SAMBROOK.** Molecular Cloning: A Laboratory Manual. Cold Spring Harbor Laboratory, 1989

- **T.J. SILHAVY ; M.L. BERMAN ; L.W. ENQUIST.** Experiments with Gene Fusions. Cold Spring Harbor Laboratory, 1984
- **AUSUBEL, F.M. et al.** Current Protocols in Molecular Biology. Greene Publishing Assoc. and Wiley Interscience, 1987
- Current Protocols in Molecular Biology. Wiley Interscience, 1997
- **SAMBROOK et al.** Molecular Cloning: A Laboratory Manual. Cold Spring Harbor Laboratory Press, 1989
- **THOMAS, K.R. ; CAPECCHI, M.R.** *Cell,* 1987, vol. 51, 503
- **T. MANIATIS ; E.F. FRITSCH ; J. SAMBROOK.** Molecular Cloning: A Laboratory Manual. Cold Spring Harbor Laboratory, 1989
- **HARLOW, E. ; LANE, D.** Antibodies: A Laboratory Manual. Cold Spring Harbor (N.Y.) Press, 1988
- Biotechnology. 1993, vol. 3